# EUROPEAN PATENT APPLICATION

(11) **EP 2 829 303 A1**
(43) Date of publication of application: **28.01.2015**
(21) Application number: 14177903.3
(22) Date of filing: 21.07.2014
(51) Int. Cl.: A61Q 19/00, A61K 8/97, A61Q 19/02, A61Q 19/08

(54) **Compositions comprising extract of malva neglecta and method of treating a skin condition with malva neglecta**

(30) Priority: 22.07.2013 US 201313947489; 22.07.2013 US 201313947473
(71) Applicant: Johnson & Johnson Consumer Companies Inc., Skillman, NJ 08558 (US)
(72) Inventor: Southall, Michael D., Pennington, NJ New Jersey 08534 (US); Mahmood, Khalid, South Hadley, MA Massachusetts 01075 (US); Pappas, Apostolos, Bridgewater, NJ New Jersey 08807 (US); Hu, Yaping, Somerset, NJ New Jersey 08873 (US); Reynertson, Kurt, Hopewell, NJ New Jersey 08525 (US); Chon, Suhyoun, Princeton, NJ New Jersey 08540 (US); Parsa, Ramine, Lawrenceville, NJ New Jersey 08648 (US); Randhawa, Manpreet, Plainsboro, NJ New Jersey 08536 (US)
(74) Representative: Carpmaels & Ransford LLP

(57) **Abstract**

The present invention relates to a skin care composition comprising an extract of *Malva neglecta* and a cosmetically acceptable topical carrier. Non-polar and / or lipophilic extracts have been found to be particularly effective in improving skin barrier function, improving the appearance of at least one sign of aging in skin, and / or lightening skin. The composition may further comprise an agent, such as an active cosmetic agent, for further treating the skin condition. The present invention also relates to a method of treating a skin condition, particularly improving skin barrier function, improving the appearance of at least one sign of aging in skin, and / or lightening skin, by applying to the skin an extract of *Malva neglecta* topically to the skin in need of treatment. Topical application of non-polar and / or lipophilic extracts of *Malva neglecta* have been found to be particularly effective in improving skin barrier function, improving the appearance of at least one sign of aging in skin, and / or lightening skin. The extract may be applied in a composition further comprising an agent, such as an active cosmetic agent, for further treating the skin condition.

## Description

### FIELD OF INVENTION

The present invention relates to compositions comprising plant extracts for use on skin. More specifically, the present invention relates to compositions comprising extracts of *Malva neglecta* for improving the condition and appearance of the skin, such as by enhancing skin barrier protection, improving, reducing, inhibiting or delaying the appearance of at least one sign of aging in skin, and / or lightening skin.

The present invention also relates to a method of using compositions comprising plant extracts to treat a skin condition. More specifically, the present invention relates to a method of using compositions comprising extracts of *Malva neglecta* for improving the condition and appearance of the skin, such as by enhancing skin barrier protection, improving, reducing, inhibiting or delaying the appearance of at least one sign of aging in skin, and / or lightening skin.

### DESCRIPTION OF RELATED ART

*Malva neglecta* is typically designated a "weed." Native to the "Old World," it has been naturalized throughout North America. *Malva neglecta* is native to almost all of Europe, from northern Europe (e.g., Denmark, Ireland, Norway, Sweden, United Kingdom), middle Europe *(e.g.,* Austria, Belgium), Southeastern Europe *(e.g.,* Albania, Bulgaria, Croatia, etc.), to Southwestern Europe (e.g., France, Portugal, Spain). It is also found in Western Asia, the Arabian Peninsula, Northwestern Asia (e.g., Armenia, Georgia, Kazakhstan, Uzbekistan, Mongolia) and also in China and the Indian subcontinent. In Africa, it is found mostly in North Africa, such as Algeria and Morocco.

Many *Malva* species are used in traditional medicinal systems around the world, including *Malva neglecta.* It has also been commonly used as a food. It has not been commercialized as a trade herb. It is also known by various common names - Common mallow, buttonweed, cheeseplant, cheeseweed, dwarf mallow, and roundleaf mallow.

According to Plants For A Future (http://www.pfaf.org/user/default.aspx), the online database for medicinal and edible wild plants, *Malva neglecta* is described for use as anti-inflammatory, anti-phlogistic, astringent, demulcent, diuretic, emollient, expectorant, laxative, poultice, purgative, and salve.

The uses of *Malva neglecta* disclosed in Plants For A Future, as well as other known uses of *Malva neglecta* described in other references, are mostly in ingested forms, except for use as an emollient or salve or poultice. Some traditional literature also describes the use of a poultice for eczema.

Plants or botanicals may be formed into compositions for topical application in a variety of manners. A poultice is a soft moist mass, often heated and medicated, that is spread on cloth over the skin to treat an aching, inflamed, or painful part of the body. It can be used on wounds such as cuts. A decoction involves boiling plant material in water to extract certain chemicals or properties. An infusion is prepared by steeping plant material in hot water (like a tea bag). A solvent-based extraction is made by grinding or macerating plant material in a solvent, typically an organic solvent such as an alcohol, acetone, hexane, or chloroform. Typical traditional methods of forming compositions from plants or botanicals, such as described in Plants For A Future and the other prior art references generally employ poultice or decoction or infusion methods of preparation. In particular, traditional art describes use of *Malva neglecta* in forms such as a water decoction, after removing insoluble parts of the plant taken orally, as a poultice, or an infusion applied to burns, insect bites, and wounds. By using water, these methods typically extract only the most polar constituents, e.g., tannins.

Topical uses of *Malva neglecta* reported in the prior art (S. Foster and JA Duke, Medicinal Plants and Herbs, pp. 170-171, New York, Houghton Mifflin Company 2000) are limited to wounds and tumors. However, more common species of *Malva* genus, *e.g., Malva sylvestris,* are sometimes also extended to *Malva neglecta* in the form of decoctions or compresses for treating abscesses, boils, burns, eczema, and insect bites. (E. Launert, The Hamlyn Guide to Edible & Medicinal Plants, p.50; D. Bown, New Encyclopedia of Herbs and Their Uses, pp.270-271, New York, DK Publishing, Inc. 2001). Traditional literature also describes the preparation of *Malva neglecta* as a poultice or decoction for medicinal uses described above. Poultices and decoctions are obtained when raw materials are soaked in water with or without heat and may or may not involve separation of plant materials before application. According to this description of preparing *Malva neglecta* for therapeutic purposes, it is obvious that the most effective preparation would be such where hydrophilic components, e.g., tannins, are extracted in water, more so in boiling water, such as described in E. Launert, Edible & Medicinal Plants. Tannins are naturally occurring plant polyphenols and are hydrophilic components with astringent taste.

### SUMMARY OF THE INVENTION

The present invention relates to applicants' discovery that certain extracts of *Malva neglecta* are beneficial for use in topical compositions for application to the skin and provide significant and unexpected benefits for skin, including enhancing skin barrier protection and skin moisturization, improving, reducing, inhibiting, or delaying the appearance of at least one sign of aging in skin (hereinafter referenced as just "improving the appearance of at least one sign of aging" for the sake of simplicity, the term "improving" to be understood to include reducing, inhibiting, delaying, and the like), and lightening the skin. In particular, applicants have discovered that a topical composition made from *Malva neglecta* following the traditional methods of preparation does not produce a positive result for improving skin barrier function and for improving moisturization in the skin, or affecting at least one sign of aging in skin or skin pigmentation.

Surprisingly, it was discovered that an effective *Malva neglecta* extract for skin moisturization, improving skin barrier function, improving the appearance of at least one sign of aging in skin, and affecting skin pigmentation uses non-polar solvents. Non-polar solvents may include a solvent selected from the group consisting of liquid carbon dioxide with or without polarity modifier, aqueous ethanol, C₁-C₈ alcohols (such as methanol, ethanol, propanols, and butanols), C₁-C₈ alkanes (such as pentanes, hexanes, and heptanes), C₂-C₈ glycols / polyols (such as glycerine, butylene glycols, and propylene glycols), C₅-C₈ cycloalkanes (such as cyclopentanes, cyclohexanes, and cycloheptanes), C₁-C₈ alkyl ethers, C₁-C₈ aliphatics, ketones, methylene chloride, ethyl acetate, xylene, toluene, vegetable oil, mineral oil, and combinations thereof.

Surprisingly, it was also discovered that an effective *Malva neglecta* extract for skin moisturization, improving skin barrier function, improving the appearance of at least one sign of aging in skin, and affecting skin pigmentation uses a lipophilic extract of *Malva neglecta.*

The present invention therefore provides a composition of a non-polar, or lipophilic, or non-polar lipophilic extract of *Malva neglecta,* a cosmetically acceptable topical carrier, and an active agent for improving skin barrier function.

The present invention therefore provides a composition of a non-polar, or lipophilic, or non-polar lipophilic extract of *Malva neglecta,* a cosmetically acceptable topical carrier, and an active agent for improving the appearance of at least one sign of aging in skin.

The present invention therefore provides a composition of a non-polar, or lipophilic, or non-polar lipophilic extract of *Malva neglecta,* a cosmetically acceptable topical carrier, and an active agent for lightening skin.

The present invention also provides a method of improving the barrier function and moisturization of skin, comprising topically applying to skin in need of improving skin barrier function and moisturization a composition comprising an extract of *Malva neglecta.*

The present invention also provides a non-polar and / or lipophilic extract of *Malva neglecta* for use in improving the barrier function of skin. The extract may be formulated in a topical composition for application to skin, *e.g.* skin in need of improving skin barrier function.

The present invention also provides a cosmetic method of improving the barrier function of skin, said method comprising applying a topical composition of a non-polar and / or lipophilic extract of *Malva neglecta* to skin in need of improving skin barrier function to result in improved skin barrier function.

In addition, the present invention provides a method of improving the appearance of at least one sign of aging in skin, comprising topically applying to skin in need of treatment for at least one sign of skin aging a composition comprising an extract of *Malva neglecta.*

The present invention also provides a non-polar and / or lipophilic extract of *Malva neglecta* for use in improving the appearance of at least one sign of skin aging. The extract may be formulated in a topical composition for application to skin, *e.g.* skin in need of improving the appearance of at least one sign of aging.

The present invention also provides a cosmetic method of improving the appearance of at least one sign of skin aging, said method comprising applying a topical composition of a non-polar and / or lipophilic extract of *Malva neglecta* to skin in need of improving the appearance of at least one sign of aging in skin therein to result in improvement of the appearance of at least one sign of aging in the skin

The present invention further provides a method for lightening the skin, comprising topically applying to skin in need of skin lightening treatment a composition comprising an extract of *Malva neglecta.*

The present invention also provides a non-polar and / or lipophilic extract of *Malva neglecta* for use in lightening skin. The extract may be formulated in a topical composition for application to skin, *e.g.* skin in need of lightening.

The present invention also provides a cosmetic method of lightening skin, said method comprising applying a topical composition of a non-polar and / or lipophilic extract of *Malva neglecta* to skin in need of lightening to result in lightened skin.

The extract of *Malva neglecta* to be applied topically to skin to treat a skin condition preferably is a non-polar and / or lipophilic extract of any part of the *Malva neglecta* plant.

The topical composition - of the present invention preferably includes a cosmetically acceptable topical carrier as well. The topical carrier may include active ingredients for treating a skin condition that the *Malva neglecta* is being used to treat.

The topical composition of *Malva neglecta* to be applied in accordance with principles of the present invention preferably is made as a result of a solvent based extraction, and contains a wider range of non-polar compounds than in the traditional topical compositions of the prior art. Preferably, the plant biomass is separated entirely from the extraction, and is not used after extraction.

### DESCRIPTION OF THE INVENTION

All percentages listed in this specification, unless otherwise stated, are weight percentages based on the total weight of composition.

As used herein, "skin in need of improving skin barrier function and moisturization" means a skin that is, but not limited to, lacking in moisture, lacking in sebum, cracked, dry, itchy, scaly, xerodermic, dehydrated, lacks suppleness, lacks radiance, dull or lacks lipids.

As used herein, "skin in need of treatment for at least one sign of skin aging" means a skin that is, but not limited to, sagging, loose, lax, rough, wrinkly, thinned, or uneven. Improving the appearance of a sign of skin aging means improving the firmness of skin, improving the texture of skin, improving the appearance of wrinkles in skin, improving the skin tone, or treating external aggressions in skin. As noted above, "improving" is to be understood as including reducing, inhibiting, and / or delaying, and the like.

As used herein, "improving the firmness of skin" means the enhancing of the firmness or elasticity of the skin, preventing the loss of firmness or elasticity of skin, or preventing or treating sagging, lax and loose skin. The firmness or elasticity of the skin can be measured by use of a cutometer. See Handbook Of Non-Invasive Methods And The Skin, eds. J. Serup, G. Jemec & G. Grove, Chapter 66.1 (2006). The loss of skin elasticity or firmness may be a result of a number of factors, including but not limited to aging, environmental damage, or the result of an application of a cosmetic to the skin.

As used herein, "improving the texture of skin" means the smoothing of the surface of the skin to remove either bumps or crevasses on the skin surface.

As used herein, "improving the appearance of wrinkles in skin" means preventing, retarding, arresting, or reversing the process of wrinkle and fine line formation in skin.

As used herein, "treating external aggressions in skin" means the reduction or prevention of the damage from external aggressions in skin. Examples of external aggressions include, but are not limited to, damage to the skin from the use of cleansers (e.g., topical cleansers containing surfactants), make-up, shaving as well as environmental damage such as from UV light *(e.g.,* sun damage from sunlight or damage from non-natural sources such as UV lamps and solar simulators), ozone, exhaust, pollution, chlorine and chlorine containing compounds, and cigarette smoke. Effects of external aggressions on the skin include, but are not limited to, oxidative and/or nitrosative damage to and modifications on lipids, carbohydrates, peptides, proteins, nucleic acids, and vitamins. Effects of external aggressions on the skin also include, but are not limited to, loss of cell viability, loss or alteration of cell functions, and changes in gene and/or protein expression.

As used herein, "improving the skin tone" means the lightening of the appearance of the skin (e.g., lightening pigmented marks or lesions, reducing skin sallowness, and/or evening the color of the skin).

As used herein, the term "lightening the appearance of skin" refers generally to lightening, brightening, whitening, and/or evening of the skin tone, skin color, and/or shade of skin, and/or to the reduction in sallowness, and/or to the lightening and/or fading of hyperpigmented marks and/or lesions including, but not limited to, pigmented spots, melanin spots, age spots, sun spots, senile lentigos, freckles, lentigos simplex, pigmented solar keratosis, seborrhoeic keratosis, melasma, acne marks, post-inflammatory hyperpigmentation, lentigines, ephelides, combinations of two or more thereof and the like. In certain embodiments, "lightening the appearance of skin" also refers to increased skin radiance, glow, translucency and/or luminescence and/or obtaining a more radiant, glowing, translucent or luminous skin tone appearance or a less yellow or sallow skin tone. In certain preferred embodiments, "lightening the appearance of skin" refers to lightening and evening the skin tone, increasing skin radiance and/or lightening age spots.

As used herein, the term "skin in need of skin lightening treatment" refers generally to skin that exhibits one or more property selected from the group consisting of: skin having a measured Individual Typology Angle (ITA) value below 41 as determined per the COLIPA GUIDELINE: GUIDELINE FOR THE COLORIMETRIC DETERMINATION OF SKIN COLOUR TYPING AND PREDICTION OF THE MINIMAL ERYTHEMAL DOSE (MED) WITHOUT UV EXPOSURE published in 2007, which is incorporated herein by reference and further described below, darkened and/or sallow skin, including skin darkened by UV, skin with uneven skin tone, or skin with one or more hyperpigmented marks and/or lesions including, but not limited to, pigmented spots, melanin spots, age spots, sun spots, senile lentigos, freckles, lentigos simplex, pigmented solar keratosis, seborrhoeic keratosis, melasma, acne marks, post-inflammatory hyperpigmentation, lentigines, ephelides, combinations of two or more thereof and the like. In the COLIPA guidelines, skin color is defined function of the ITA value as: very light skin >55; Light skin 41-55, Intermediate 28-41, and Tan skin <28. In certain preferred embodiments, "skin in need of skin lightening" refers to individuals with a skin having an ITA value of less than 41, such as about 40 or less, about 35 or less, about 30 or less, or more preferably about 28 or less. In certain other preferred embodiments, the present invention is directed to compositions and methods for use on skin in need of skin lightening treatment selected from sallow and/or darkened skin. In certain other preferred embodiments, the present invention is directed to compositions and methods for use on skin in need of skin lightening treatment selected from the group consisting of age spots, freckles, marks left after acne, and combinations of two or more thereof

As used herein, "cosmetically / dermatologically acceptable" means suitable for use in contact with tissues *(e.g.,* the skin or hair) without undue toxicity, incompatibility, instability, irritation, allergic response, and the like.

As used herein, the term "safe and effective amount" means an amount sufficient to induce the desired effect, but low enough to avoid serious side effects. The safe and effective amount of the compound, extract, or composition will vary with, *e.g.,* the age, health and environmental exposure of the end user, the duration and nature of the treatment, the specific extract, ingredient, or composition employed, the particular pharmaceutically-acceptable carrier utilized, and like factors.

As described herein, applicants have discovered that extracts of *Malva neglecta* and topical compositions containing them provide unexpectedly good skin barrier function, skin moisturization, skin anti-aging, and skin lightening benefits.

In particular, applicants have discovered that certain extracts of *Malva neglecta* provide a significant increase in ceramide levels in human skin cells, which is indicative of improved skin barrier function. An improved skin barrier function is desirable for an overall skin health and specifically for improving the appearance of at least one sign of aging. Roger, et al. (Rogers J, Harding C, Mayo A, Banks J, Rawlings A. Stratum corneum lipids: the effect of ageing and the seasons, Arch Dermatol Res. 1996 Nov; 288(12):765-70) demonstrated significant decreased levels of major skin lipid species, in particular ceramides with increasing age. Independently, Jensen, et al (Jensen JM, Förl M, Winoto-Morbach S, Seite S, Schunck M, Proksch E, Schütze S., Exp Dermatol Exp Dermatol., Acid and neutral sphingomyelinase, ceramide synthase, and acid ceramidase activities in cutaneous aging, 2005 Aug;14(8):609-18.) demonstrated reduced activities of ceramide-generating enzymes in the inner epidermis of aged skin. Taken together, it is highly desirable to increase skin lipid levels, e.g., ceramide levels, to achieve significant improvements in skin barrier structure and function specifically improving on the appearance of at least one sign of aging.

Applicants have also discovered that topical application of certain extracts of *Malva neglecta* enhance the endogenous hyaluronic acid ("HA") levels, indicating improvements in the appearance of at least one sign of skin aging. According to: Tzellos T. G., Klagas I., Vahtsevanos K., Triaridis S., Printza A., Kyrgidis A., Karakiulakis G., Zouboulis C. C., and Papakonstantinou E., "Extrinsic Aging in the Human Skin Is Associated With Alterations in the Expression of Hyaluronic Acid and Its Metabolizing Enzymes," Experimental Dermatology 18, No. 12 (2009), in addition to normal intrinsic skin aging, also in "extrinsic skin aging" or "photoaging"' (as a result of exposure to external factors, mainly ultraviolet irradiation), the expression of hyaluronic acid producing genes decrease and that of hyaluronic acid degrading genes increase. According to Sidgwick G.P., Ingbal S.A., and Bayat A., "Altered Expression of Hyaluronan Synthase and Hyaluronidase MRNA May Affect Hyaluronic Acid Distribution in Keloid Disease Compared with Normal Skin," Experimental Dermatology 22, No. 5 (2013), in Keloid disease ("KD"), a fibroproliferative skin disorder characterized partly by an altered extracellular matrix ("ECM") profile, the hyaluronic acid ("HA") expression is reduced in KD tissue compared with normal skin (NS). Dry, scaly skin is frequently seen in the elderly. The degradation or loss of skin barrier function with increasing age is partly accountable for this manifestation. The recovery of damaged barrier function has been demonstrated to be slower in aged skin, resulting in greater susceptibility to developing dryness. This is a multifactorial process due, in part, to lower lipid levels in lamellar bodies (according to "The Aged Epidermal Permeability Barrier. Structural, Functional, And Lipid Biochemical Abnormalities In Humans And A Senescent Murine Model," J. Clin. Invest. 1995; 95(5):2281-2290), and a decrease in epidermal filaggrin (according to "Terminal Differentiation Of Facial Epidermis Of The Aged: Immunohistochemical Studies," Dermatology, 1994;188(1):21-24). Increased transepidermal water loss (TEWL) is also exhibited by aged skin, leaving the stratum corneum more susceptible to becoming dry. Also, the formation of wrinkles is considered the most conspicuous and common manifestation, and nearly a sine qua non feature, of skin aging. To prevent the formation of wrinkles, it is necessary to halt the degradation of the skin's three primary structural constituents, collagen, elastin, and HA. HA can bind 1000 times its weight in water, and may help the skin retain and maintain water. HA is found in young skin at the periphery of collagen and elastin fibres and where these types of fibres intersect. In aged skin, such connections with HA disappear (according to Ghersetich I, Lotti T, Campanile G, Grappone C, Dini G., "Hyaluronic acid in cutaneous intrinsic aging," Int J Dermatol 1994; 33(2):119-122). It is presumed that the decreases in HA levels, which contribute to its disassociation with collagen and elastin as well as reduced water binding, may be involved in the changes noted in aged skin, including wrinkling, altered elasticity, reduced turgidity and diminished capacity to support the microvasculature of the skin. Thus, increasing the levels of hyaluronic acid in skin is highly desirable. Applicants have found that certain extracts of *Malva neglecta* can increase the levels of hyaluronic acid to a direction of levels found in younger skin thereby providing the structural support to skin to reduce the appearance of at least one sign of aging in skin.

Applicants have also discovered that topical application of certain extracts of *Malva neglecta* decrease melanin production / accumulation in cells, thus indicating the extracts of *Malva neglecta* can help lighten the color of skin with high melanin content or lighten the color of dark/age/skin spots. Melanin production and accumulation correlate strongly with dark skin tone as well as some skin / age spots. Applicants measured inhibition of melanin synthesis in murine cells and also in 3D-skin equivalent tissues with *Malva neglecta* extract and found the extract to be an effective inhibitor of melanin synthesis. As shown in the Examples, the present extracts from *Malva neglecta* provide significantly superior benefits in improving the skin barrier function, moisturization, anti-aging, and skin lightening benefits compared not only to extracts from other select species of genus *Malva,* but also from other types of extracts from *Malva neglecta.*

Any suitable manner of preparing the extracts of *Malva neglecta* for use in accordance with the present invention may be used. Suitable extracts may be obtained using conventional methods including, but not limited to, direct extraction from the biomass by grinding, macerating, pressing, squeezing, mashing, centrifuging, and/or processes such as cold percolation, agitation/distillation, microwave assisted extraction, sonication, supercritical/subcritical CO₂ compressed gas extraction with or without polarity modifier, pressurized solvent extraction, accelerated solvent extraction, surfactant assisted pressurized hot water extraction, oil extraction, membrane extraction, Soxhlet extraction, the gold finger distillation/extraction and/or processes disclosed, for example, in US Pat. Nos. 7,442,391, 7,473,435, and 7,537,791 to Integrated Botanical Technologies, LLC, incorporated herein by reference, and the like, or by other methods such as solvent extraction, and the like. In particular, an extract in accordance with the present invention preferably is a solvent-based extraction made by grinding or macerating plant material in a solvent, typically an organic solvent such as an alcohol, acetone, liquid carbon dioxide with or without polarity modifier, hexane, or chloroform. The resulting extract comprised mainly non-polar compounds. The plant biomass preferably is separated entirely from the extraction, and is not used after extraction.

Any of a variety of solvents including aqueous ethanol, liquid carbon dioxide with or without polarity modifier, organic solvents, or combinations of two or more thereof may be used in methods of comprising solvent extraction. Preferably, non-polar organic solvents are used. Suitable non-polar organic solvents are C₁-C₈ alkanes, and, in particular, hexane; C₅-C₈ cycloalkanes; liquid carbon dioxide, C₁-C₈ alcohols, C₂-C₈ glycols / polyols, C₁-C₈ alkyl ethers, in particular, ethyl ether, and petroleum ethers; ketones, including C₃-C₈ ketones, methylene chloride, ethyl acetate, xylene, toluene, chloroform, vegetable oil, mineral oil and the like. Particularly effective, and thus preferred solvents include aqueous ethanol, liquid carbon dioxide, vegetable oil, C₁-C₈ alcohols, C₁-C₈ alkanes, C₂-C₈ glycols / polyols, C₅-C₈ cycloalkanes, and combinations thereof. In certain embodiments, the non-polar extract is extracted from *Malva neglecta* roots using hexane, glycerine, C₃-C₄ glycols, ethanol, liquid carbon dioxide with or without polarity modifier, chloroform, or a combination thereof. In certain preferred embodiments, the non-polar extract is extracted from *Malva neglecta* roots using hexanes, ethanol, aqueous ethanol, or liquid carbon dioxide with or without polarity modifier. In certain embodiments, the non-polar extract is extracted from *Malva neglecta* aerial parts (above-ground parts, e.g., leaves, flowers, shoots, seeds, etc.) using hexane, glycerine, C₃-C₄ glycols, ethanol, aqueous ethanol, liquid carbon dioxide with or without polarity modifier, chloroform, or a combination thereof. In certain preferred embodiments, the non-polar extract is extracted from *Malva neglecta* aerial parts (above-ground parts, *e.g.,* leaves, flowers, shoots, seeds, etc.) using hexanes, ethanol, aqueous ethanol, or liquid carbon dioxide with or without polarity modifier. In certain embodiments, the non-polar extract is extracted from *Malva neglecta* whole herb using hexane, glycerine, C₃-C₄ glycols, ethanol, aqueous ethanol, liquid carbon dioxide with or without polarity modifier, chloroform, or a combination thereof. In certain preferred embodiments, the non-polar extract is extracted from *Malva neglecta* whole herb using hexanes, ethanol, aqueous ethanol, or liquid carbon dioxide with or without polarity modifier. It will be appreciated that non-polar extracts or compounds are not characterized by a dipole, and are extracts that are not ionizing when dissolved in water, a nonionic substance. A non-polar compound can also be defined as a compound comprised of molecules linked through chemical bonds arranged in such a way that the distribution of charges is symmetrical. Non-polar compounds may dissolve in water but would not dissociate into ions, e.g., non-polar amino acids.

In certain preferred embodiments, the extract of the invention is an extract prepared by pulverizing the *Malva neglecta* raw material and extracting using a solvent having a dielectric constant of a value between about 1 and about 80 at 20°C, preferably a dielectric constant of a value between about 2 and about 60 at 20°C, more preferably a dielectric constant of a value between about 2 and about 40 at 20°C, and even more preferably a dielectric constant of a value between about 2 and 35 at 20°C.

Applicants have further discovered that lipophilic extracts of *Malva neglecta* and topical compositions containing lipophilic extracts of *Malva neglecta* provide unexpectedly good skin barrier function, skin moisturization, improvement of at least the appearance of at least one sign of skin aging, and skin lightening benefits. Such extracts typically are comprised of lipids from the *Malva neglecta* plant and are freely soluble and / or extracted with fats, oils, lipids, or solvents such as alkanes, toluene, petroleum ether, or liquid CO₂ with or without polarity modifier. It will be appreciated that lipophilic extracts or compounds are generally not soluble in water and are compounds having an affinity for, tending to combine with, or capable of dissolving in lipids. Lipophilicity, hydrophobicity, and non-polarity can describe the same tendency towards participation in the London dispersion force as the terms are often used interchangeably. However, the terms "lipophilic" and "hydrophobic" are not synonymous, as can be seen with silicones and fluorocarbons, which are hydrophobic but not lipophilic. Moreover, although there is an overlap with lipophilic and non-polar extracts, such extracts can be exclusive as well. For example, non-polar amino acids are not lipophilic in nature, and free fatty acids are lipophilic compounds but are not non-polar. Sterols can be classified as both, e.g., cholesterol. An example of a solvent that results in non-polar but non-lipophilic extracts is ethyl acetate. An example of a solvent that results in lipophilic but not non-polar extracts is hexane.

In certain embodiments, the composition may include extracts from selected parts of *Malva neglecta,* for example, one or more of the leaves, shoots, roots, fruits, flowers, seeds, or flowers. In other embodiments, the composition may include extracts from the whole herb of *Malva neglecta,* including leaves, shoots, roots, fruits, flowers, and seeds. Alternatively, the composition may include an extract of the *Malva neglecta* aerial parts and/or an extract of the *Malva neglecta* roots.

Any suitable amount of extract of *Malva neglecta* may be used in the compositions of the present invention. Preferably, the compositions comprise a safe and effective amount of extract of *Malva neglecta.* In particular, the amount of *Malva neglecta* extract to be used preferably is selected to achieve the desired treatment of a given skin condition. For instance, the amount of *Malva neglecta* extract to be used to improve skin barrier function is selected based on the desired effect achieved. Likewise, the amount of *Malva neglecta* extract to be used to improve the appearance of at least one sign of aging in skin is selected to achieve the desired amount of improvement; and the amount of *Malva neglecta* extract to be used to lighten skin is selected to achieve the desired skin lightening. All such amounts are determined by applying the *Malva neglecta* extract to the skin and observing the effect until the desired results are achieved, thereby determining a therapeutically effective amount of *Malva neglecta* extract.

The efficacy of *Malva neglecta* in improving skin barrier function may be measured by an increase in ceramide levels. In one embodiment of the present invention, the amount of extract of *Malva neglecta* used in a composition of the invention is that effective to achieve an increase in the ceramide levels by at least 1% or higher, preferably about 5% or higher, and more preferably about 10% or higher according to the test Determination of Ceramide Profile by High-Performance Thin-layer Chromatography (Assay 5) described herein.

The efficacy of *Malva neglecta* in improving skin barrier function and / or improving the appearance of at least one sign of aging in skin may be measured by an increase in hyaluronic acid secretion. In one embodiment of the present invention, the amount of extract of *Malva neglecta* used in a composition of the invention is that effective for providing an increase of hyaluronic acid secretion to greater than 1.2 fold, or, more preferably, greater than 1.5 fold, and, more preferably, greater than 2.0 fold over the control, when measured in accordance with the Hyaluronic acid (HA) Secretion test (Assay 2) described herein.

The efficacy of *Malva neglecta* in providing skin lightening benefit can be measured by decrease in melanin production. In one embodiment of the present invention, the amount of extract of *Malva neglecta* used in a composition formed in accordance with principles of the present invention is an amount effective for providing decrease in melanin production to greater than 10% or greater, preferably 30% or greater, and more preferably greater than 50%, when measured in accordance with the B16 melanin assay (Assay 7).

In certain preferred embodiments, the compositions comprise from greater than zero to about 20% extract of *Malva neglecta.* In certain other preferred embodiments, the compositions comprise from about 0.0001 to about 20%, from about 0.001 to about 10%, from about 0.01 to about 5%, from about 0.1 to about 5%, or from about 0.2 to about 2% of extract of *Malva neglecta.*

Any suitable carrier may be used in the compositions. Preferably, the carrier is a cosmetically-acceptable carrier. As will be recognized by those of skill in the art, cosmetically-acceptable carriers comprise carriers that are suitable for use in contact with the body, in particular the skin, without undue toxicity, incompatibility, instability, irritation, allergic response, and the like. A safe and effective amount of carrier is from about 50% to about 99.999%, preferably from about 80% to about 99.9%, more preferably from about 99.9% to about 95%, most preferably from about 98% to about 99.8% of the composition.

The carrier can be in a wide variety of forms. For example, carriers in the form of emulsions, including, but not limited to, oil-in-water, water-in-oil, water-in-oil-in-water, and oil-in-water-in-silicone emulsions, are useful herein. These emulsions can cover a broad range of viscosities, *e.g.,* from about 100 cps to about 200,000 cps.

Examples of suitable cosmetically-acceptable carriers include cosmetically-acceptable solvents and materials for cosmetic solutions, suspensions, lotions, creams, serums, essences, gels, toners, sticks, sprays, ointments, liquid washes and soap bars, shampoos, hair conditioners, pastes, foams, mousses, powders, shaving creams, wipes, patches, strips, powered patches, microneedle patches, bandages, hydrogels, film-forming products, facial and skin masks, make-up, liquid drops, and the like. These product types may contain several types of cosmetically- acceptable carriers including, but not limited to solutions, suspensions, emulsions such as microemulsions and nanoemulsions, gels, solids, liposomes, other encapsulation technologies and the like.

The following are non-limitative examples of carriers. Other carriers can be formulated by those of ordinary skill in the art.

In one embodiment, the carrier contains water. In a further embodiment, the carrier may also contain one or more aqueous or organic solvents. Examples of organic solvents include, but are not limited to: dimethyl isosorbide; isopropylmyristate; surfactants of cationic, anionic and nonionic nature; vegetable oils; mineral oils; waxes; gums; synthetic and natural gelling agents; alkanols; glycols; and polyols. Examples of glycols include, but are not limited to, glycerin, propylene glycol, butylene glycol, pentalene glycol, hexylene glycol, polyethylene glycol, polypropylene glycol, diethylene glycol, triethylene glycol, capryl glycol, glycerol, butanediol and hexanetriol, and copolymers or mixtures thereof. Examples of alkanols include, but are not limited to, those having from about 2 carbon atoms to about 12 carbon atoms (e.g., from about 2 carbon atoms to about 4 carbon atoms), such as isopropanol and ethanol. Examples of polyols include, but are not limited to, those having from about 2 carbon atoms to about 15 carbon atoms (e.g., from about 2 carbon atoms to about 10 carbon atoms) such as propylene glycol. The organic solvents may be present in the carrier in an amount, based upon the total weight of the carrier, of from about 1 percent to about 99.99 percent (e.g., from about 20 percent to about 50 percent). Water may be present in the carrier (prior to use) in an amount, based upon the total weight of the carrier, of from about 5 percent to about 95 percent (e.g., from about 50 percent to about 90 percent). Solutions may contain any suitable amounts of solvent, including from about 40 to about 99.99%. Certain preferred solutions contain from about 50 to about 99.9%, from about 60 to about 99%, from about 70 to about 99%, from about 80 to about 99%, or from about 90 to 99% of solvent.

A lotion can be made from such a solution. Lotions typically contain at least one emollient in addition to a solvent. Lotions may comprise from about 1% to about 20% *(e.g.,* from about 5% to about 10%) of an emollient(s) and from about 50% to about 90% (e.g., from about 60% to about 80%) of water.

Another type of product that may be formulated from a solution is a cream. A cream typically contains from about 5% to about 50% *(e.g.,* from about 10% to about 20%) of an emollient(s) and from about 45% to about 85% *(e.g.,* from about 50% to about 75%) of water.

Yet another type of product that may be formulated from a solution is an ointment. An ointment may contain a simple base of animal, vegetable, or synthetic oils or semi-solid hydrocarbons. An ointment may contain from about 2% to about 10% of an emollient(s) plus from about 0.1% to about 2% of a thickening agent(s).

The compositions useful in the present invention can also be formulated as emulsions. If the carrier is an emulsion, from about 1% to about 10% *(e.g.,* from about 2% to about 5%) of the carrier contains an emulsifier(s). Emulsifiers may be nonionic, anionic or cationic.

Lotions and creams can be formulated as emulsions. Typically such lotions contain from 0.5% to about 5% of an emulsifier(s), while such creams would typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type, and water-in-oil type are well-known in the art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel *(e.g.,* an aqueous, alcohol, alcohol/water, or oil gel using a suitable gelling agent(s)). Suitable gelling agents for aqueous and/or alcoholic gels include, but are not limited to, natural gums, acrylic acid and acrylate polymers, and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

The compositions of the present invention can also be formulated into a solid formulation (e.g., a wax-based stick, soap bar composition, powder, or wipe). The composition of the present invention can also be combined with a solid, semi-solid, or dissolvable substrate *(e.g.,* a wipe, mask, pad, glove, or strip).

The compositions of the present invention may further comprise any of a variety of additional cosmetically active agents. Examples of suitable additional active agents include: skin lightening agents, darkening agents, additional anti-aging agents, tropoelastin promoters, collagen promoters, anti-acne agents, shine control agents, anti-microbial agents such as anti-yeast agents, anti-fungal, and anti-bacterial agents, anti-inflammatory agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, hair growth enhancing agents, hair growth delaying agents, firming agents, hydration boosters, efficacy boosters, anti-callous agents, agents for skin conditioning, anti-cellulite agents, odor-control agents such as odor masking or pH-changing agents, and the like.

Examples of various suitable additional cosmetically acceptable actives include hydroxy acids; benzoyl peroxide; D-panthenol; UV filters such as but not limited to avobenzone (Parsol 1789), bisdisulizole disodium (Neo Heliopan AP), diethylamino hydroxybenzoyl hexyl benzoate (Uvinul A Plus), ecamsule (Mexoryl SX), methyl anthranilate, 4-aminobenzoic acid (PABA), cinoxate, ethylhexyl triazone (Uvinul T 150), homosalate, 4-methylbenzylidene camphor (Parsol 5000), octyl methoxycinnamate (Octinoxate), octyl salicylate (Octisalate), padimate O (Escalol 507), phenylbenzimidazole sulfonic acid (Ensulizole), polysilicone-15 (Parsol SLX), trolamine salicylate, Bemotrizinol (Tinosorb S), benzophenones 1-12, dioxybenzone, drometrizole trisiloxane (Mexoryl XL), iscotrizinol (Uvasorb HEB), octocrylene, oxybenzone (Eusolex 4360), sulisobenzone, bisoctrizole (Tinosorb M), titanium dioxide, zinc oxide; carotenoids; free radical scavengers; spin traps; retinoids and retinoid precursors such as retinol, retinoic acid and retinyl palmitate; ceramides; polyunsaturated fatty acids; essential fatty acids; enzymes; enzyme inhibitors; minerals; hormones such as estrogens; steroids such as hydrocortisone; 2-dimethylaminoethanol; copper salts such as copper chloride; peptides containing copper such as Cu:Gly-His-Lys, coenzyme Q10; amino acids such a proline; vitamins; lactobionic acid; acetyl-coenzyme A; niacin; riboflavin; thiamin; ribose; electron transporters such as NADH and FADH2; and other botanical extracts such as oat, aloe vera, Feverfew, Soy, Shiitake mushroom extracts, and derivatives and mixtures thereof.

In certain preferred embodiments, the skin care compositions comprise an extract of *Malva neglecta* and at least one additional skin moisturizing active agent.

In certain preferred embodiments, the skin care compositions comprise an extract of *Malva neglecta* and at least one additional agent for improving the appearance of at least one sign of aging in skin. Examples of suitable additional agents improving the appearance of at least one sign of aging in skin include, but are not limited to, tropoelastin promoters, collagen promoters, retinoids, hyaluronic acid, dimethylaminoethanol, N,N,N',N'-tetrakis(2-hydroxypropyl)ethylenediamine, alpha hydroxy acids, polyhydroxyacids, and combinations of two or more thereof.

"Tropoelastin promoters," as used herein, refers to a class of compounds that possess the biological activity of enhancing the production of tropoelastin. Tropoelastin promoters, according to the present invention, include all natural or synthetic compounds that are capable of enhancing the production of tropoelastin in the human body.

Examples of suitable tropoelastin promoters include, but are not limited to, blackberry extracts, cotinus extracts, feverfew extracts, extracts of *Phyllanthus niruri* and bimetal complexes having copper and/or zinc constituents. The bimetal complex having copper and/or zinc constituents may be, for example, copper-zinc citrate, copper-zinc oxalate, copper-zinc tartarate, copper-zinc malate, copper-zinc succinate, copper-zinc malonate, copper-zinc maleate, copper-zinc aspartate, copper-zinc glutamate, copper-zinc glutarate, copper-zinc fumarate, copper-zinc glucarate, copper-zinc polyacrylic acid, copper-zinc adipate, copper-zinc pimelate, copper-zinc suberate, copper-zinc azealate, copper-zinc sebacate, copper-zinc dodecanoate, or combinations thereof. In a preferred embodiment, the tropoelastin promoter is selected from blackberry extracts, cotinus extracts, feverfew extracts, and combinations thereof. In a particularly preferred embodiment, the tropoelastin promoter is selected from blackberry extracts, feverfew extracts, and combinations thereof.

By "cotinus extract," it is meant an extract of the leaves of *"cotinus coggygria,*" such as a water extract thereof, available from Bilkokoop of Sofia, Bulgaria.

By "blackberry extract," it is meant a blend of compounds isolated from the plant of the genus *Rubus,* and preferably *Rubus fruticosus.* In one embodiment, the compounds are isolated from the flowers of the plant. In a further embodiment, the compounds are isolated from dried flowers of the plant. Such compounds may be isolated from one or more part of the plant *(e.g.,* the whole plant, flower, seed, root, rhizome, stem, fruit and/or leaf of the plant). In a preferred embodiment, the blackberry extract is a blackberry leaf extract. One particularly suitable blackberry extract is produced by extracting the leaves of *Rubus fruticosus* with a mixture of water and ethanol compounded to an activity of about 5% to about 10%, with a maltodextrin matrix, commercially available from Symrise Inc. of Teterboro, NJ, and is sold under the name "SymMatrix."

Extracts of "*Phyllahthus niruri*" may be harvested and used as the whole plant, or optionally one or more parts of the plant (e.g., flower, seed, root, rhizome, stem, fruit and/or leaf of the plant) may be used. The *Phyllahthus niruri* plant or parts thereof may be finely divided, such as by grinding or milling, to a powder. A suitable milled form of *Phyllanthus niruri* is commercially available from Raintree Nutrition, Inc., of Carson City, Nevada. Preferably, a low molecular weight fraction of *Phyllanthus niruri* is used, for instance a fraction of *Phyllanthus niruri* substantially free of molecular species having a molecular weight of greater than about 100,000 daltons. Preferably, such low molecular weight fraction is water extractable from the *Phyllanthus niruri* plant.

Compositions of the present invention may include a cosmetically effective amount of one or more tropoelastin promoters such as those described above. The compositions preferably include, on an active basis, from about 0.1% to about 10% of the tropoelastin promoters, more preferably from about 0.5% to about 5% of tropoelastin promoters, and most preferably from about 0.5% to about 2% of the tropoelastin promoters.

"Collagen promoter," as used herein, refers to compounds that possess the biological activity of enhancing the production of collagen. "Non-retinoid collagen promoters" according to the present invention include all natural or synthetic compounds that are not retinoids, or derived from retinoids, and are capable of enhancing the production of collagen in the human body.

Examples of suitable collagen promoters include, but are not limited to the following: Retinoids including retinol, retinaldehyde, and retinoic acid, extracts of feverfew (*Tanacetum parthenium*)*,* extracts of *Centella asiatica,* and extracts of *Siegesbeckia orientalis;* extracts of soy; collagen-promoting peptides; ursolic acid; and asiaticoside.

*Centella asiatica,* also known as *Violette marronne* on Reunion Island, Gotu Kola or Indian pennywort in India, *Centella repanda* in North America, and Talapetraka in Madagascar, is a polymorphous herb and belongs to the family of *Umbelliferae* (*Apiaceae*), particularly to the *Hydrocotyle* subfamily. It grows wild throughout the tropics and prefers moist and shady regions at an altitude of about 600 to 1200 meters above sea level. *Centella asiatica* has three varieties: Typica, Abyssinica, and Floridana. The herb is known and used for its healing, sedative, analgesic, antidepressant, antiviral and antimicrobial properties. The biological activity of the herb appears to be due to the presence of triterpene molecules in the herb. A suitable extract of *Centella asiatica* is available as TECA from Bayer Consumer HealthCare of Basel, Switzerland.

By "extracts of *Siegesbeckia orientalis,"* is meant any of various extracts of the plant *Siegesbeckia orientalis,* including Darutoside available from Sederma (Croda International Group of Edison, NJ).

Suitable collagen-promoting peptides include the following matrikine peptides, (*i.e.,* a peptide derived from the degradation of extracellular matrix proteins - collagen, elastin, or proteoglycan) including palmitoyl pentapeptides, in particular Pal-Lys-Thr-Thr-Lys-Ser-OH, available as MATRIXYL from Sederma (Croda International Group of Edison, NJ); GHK copper peptide available as PROCYTE from Photomedex of Montgomeryville, PA;Palmitoyl GHK peptide available as Biopoeptide CL from Sederma (Croda International Group of Edison, NJ); Biomimetic tetrapeptides, such as those available as Chronoline Tri Peptide from Unipex of Quebec, Canada ; and Palmitoyl tri-peptide, available as Syn-Coll from DSM of Basel, Switzerland.

Ursolic acid is also known as pentacyclic triterpene acid, Prunol, Malol, Urson, beta-ursolic acid and 3-Beta-Hydroxy-Urs-12-En-28-Oic Acid. It is commercially available for example from Sigma-Aldrich of St. Louis, MO.

Asiaticoside, also known chemically as: [6-[[3,4-dihydroxy-6-(hydroxymethyl)-5-(3,4,5-trihydroxy-6-methyloxan-2-yl)oxyoxan-2-yl]oxymethyl]-3,4,5-trihydroxyoxan-2-yl] 10,11-dihydroxy-9-(hydroxymethyl)-1,2,6a,6b,9,12a-hexamethyl-2,3,4,5,6,6a,7,8,8a,10,11,12,13,14b-tetradecahydro-1H-picene-4a-carboxylate) is commercially available for example from Bayer Santé Familiale Division Serdex, 69, Boulevard Victor Hugo 93400 SAINT-OUEN France.

Compositions of the present invention may include a cosmetically effective amount of one or more collagen promoters. The compositions preferably include, on an active basis, from about 0.1% to about 10% of the collagen promoters, more preferably from about 0.5% to about 5% of collagen promoters, and most preferably from about 0.5% to about 2% of the collagen promoters.

The compositions of the present invention may comprise additionally at least one skin lightening active agent. Examples of suitable skin lightening active agents include, but are not limited to, tyrosinase inhibitors, melanin-degradation agents, melanosome transfer inhibiting agents including PAR-2 antagonists, exfoliants, sunscreens, retinoids, antioxidants, Tranexamic acid, tranexamic acid cetyl ester hydrochloride, skin bleaching agents, linoleic acid, adenosine monophosphate disodium salt, Chamomilla extract, allantoin, opacifiers, talcs and silicas, zinc salts, and the like, and other agents as described in Solano et al. Pigment Cell Res. 19 (550-571) and Ando et al. Int J Mol Sci 11 (2566-2575).

Examples of suitable tyrosinase inhibitors include but, are not limited to, Vitamin C and its derivatives, Vitamin E and its derivatives, Kojic Acid, Arbutin, resorcinols, hydroquinone, Flavones e.g. Licorice flavanoids, Licorice root extract, Mulberry root extract, Dioscorea Coposita root extract, Saxifraga extract and the like, Ellagic acid, Salicylates and derivatives, Glucosamine and derivatives, Fullerene, Hinokitiol, Dioic acid, Acetyl glucosamine, 5,5'-dipropyl-biphenyl-2,2'-diol (Magnolignan), 4-(4-hydroxyphenyl)-2-butanol (4-HPB), combinations of two or more thereof, and the like. Examples of vitamin C derivatives include, but are not limited to, ascorbic acid and salts, Ascorbic Acid-2-Glucoside, sodium ascorbyl phosphate, magnesium ascorbyl phosphate, and natural extract enriched in vitamin C. Examples of vitamin E derivatives include, but are not limited to, alpha-tocopherol, beta, tocopherol, gamma-tocopherol, delta-tocopherol, alpha-tocotrienol, beta-tocotrienol, gamma-tocotrienol, delta-tocotrienol and mixtures thereof, tocopherol acetate, tocopherol phosphate and natural extracts enriched in vitamin E derivatives. Examples of resorcinol derivatives include, but are not limited to, resorcinol, 4-substituted resorcinols like 4-alkylresorcinols such as 4-butyresorcinol (rucinol), 4-hexylresorcinol (Synovea HR, Sytheon), phenylethyl resorcinol (Symwhite, Symrise), 1-(2,4-dihydroxyphenyl)-3-(2,4-dimethoxy-3-methylphenyl)-Propane (nivitol, Unigen) and the like and natural extracts enriched in resorcinols. Examples of salicylates include, but are not limited to, 4-methoxy potassium salicylate, salicylic acid, acetylsalicylic acid, 4-methoxysalicylic acid and their salts. In certain preferred embodiments, the tyrosinase inhibitors include a 4-substituted resorcinol, a vitamin C derivative, or a vitamin E derivative. In more preferred embodiments, the tyrosinase inhibitor comprises Phenylethyl resorcinol, 4-hexyl resorcinol, or ascorbyl-2-glucoside.

Examples of suitable melanin-degradation agents include, but are not limited to, peroxides and enzymes such as peroxidases and ligninases. In certain preferred embodiments, the melanin-inhibiting agents include a peroxide or a ligninase.

Examples of suitable melanosome transfer inhibiting agents including PAR-2 antagonists such as soy trypsin inhibitor or Bowman-Birk Inhibitor, Vitamin B3 and derivatives such as Niacinamide, Essential soy, Whole Soy, Soy extract. In certain preferred embodiments, the melanosome transfer inhibiting agents includes a soy extract or niacinamide.

Examples of exfoliants include, but are not limited to, alpha-hydroxy acids such as lactic acid, glycolic acid, malic acid, tartaric acid, citric acid, or any combination of any of the foregoing, beta-hydroxy acids such as salicylic acid, polyhydroxy acids such as lactobionic acid and gluconic acid, and mechanical exfoliation such as microdermabrasion. In certain preferred embodiments, the exfoliants include glycolic acid or salicylic acid.

Examples of sunscreens include, but are not limited to, avobenzone (Parsol 1789), bisdisulizole disodium (Neo Heliopan AP), diethylamino hydroxybenzoyl hexyl benzoate (Uvinul A Plus), ecamsule (Mexoryl SX), methyl anthranilate, 4-aminobenzoic acid (PABA), cinoxate, ethylhexyl triazone (Uvinul T 150), homosalate, 4-methylbenzylidene camphor (Parsol 5000), octyl methoxycinnamate (Octinoxate), octyl salicylate (Octisalate), padimate O (Escalol 507), phenylbenzimidazole sulfonic acid (Ensulizole), polysilicone-15 (Parsol SLX), trolamine salicylate, Bemotrizinol (Tinosorb S), benzophenones 1-12, dioxybenzone, drometrizole trisiloxane (Mexoryl XL), iscotrizinol (Uvasorb HEB), octocrylene, oxybenzone (Eusolex 4360), sulisobenzone, bisoctrizole (Tinosorb M), titanium dioxide, zinc oxide, and the like.

Examples of retinoids include, but are not limited to, retinol (Vitamin A alcohol), retinal (Vitamin A aldehyde), retinyl acetate, retinyl propionate, retinyl linoleate, retinoic acid, retinyl palmitate, isotretinoin, tazarotene, bexarotene, Adapalene, combinations of two or more thereof and the like. In certain preferred embodiments, the retinoid is selected from the group consisting of retinol, retinal, retinyl acetate, retinyl propionate, retinyl linoleate, and combinations of two or more thereof. In certain more preferred embodiments, the retinoid is retinol.

Examples of antioxidants include, but are not limited to, water-soluble antioxidants such as sulfhydryl compounds and their derivatives (e.g., sodium metabisulfite and N-acetylcysteine, glutathione), lipoic acid and dihydrolipoic acid, stilbenoids such as resveratrol and derivatives, lactoferrin, iron and copper chelators and ascorbic acid and ascorbic acid derivatives (e.g., ascobyl-2-glucoside, ascorbyl palmitate and ascorbyl polypeptide). Oil-soluble antioxidants suitable for use in the compositions of this invention include, but are not limited to, butylated hydroxytoluene, retinoids *(e.g.,* retinol and retinyl palmitate), tocopherols *(e.g.,* tocopherol acetate), tocotrienols, and ubiquinones. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, black tea, white tea, pine bark, feverfew, parthenolide-free feverfew, oat extracts, blackberry extract, cotinus extract, soy extract, pomelo extract, wheat germ extract, Hesperedin, Grape extract, Portulaca extract, Licochalcone, chalcone, 2,2'-dihydroxy chalcone, Primula extract, propolis, and the like.

The additional cosmetically active agent may be present in a composition in any suitable amount, for example, in an amount of from about 0.0001% to about 20% by weight of the composition, e.g., about 0.001% to about 10% such as about 0.01% to about 5%. In certain preferred embodiments, in an amount of 0.1% to 5% and in other preferred embodiments from 1% to 2%.

Compositions of the present invention may include a cosmetically effective amount of one or more anti-inflammatory compounds.

Examples of suitable anti-inflammatory agents include substituted resorcinols, (E)-3-(4-methylphenylsulfonyl)-2-propenenitrile (such as "Bay 11-7082," commercially available from Sigma-Aldrich of St. Louis, Missouri), tetrahydrocurcuminoids (such as Tetrahydrocurcuminoid CG, available from Sabinsa Corporation of Piscataway, NJ), extracts and materials derived from the following: *Phellodendron amurense* Cortex Extract (PCE), Non-Denatured Soy (*Glycine max*)*,* Feverfew (*Tanacetum parthenium),* Ginger (*Zingiber officinale),* Ginko (*Ginkgo biloba*), Madecassoside (*Centella asiatica* extract ingredient), Cotinus (*Cotinus coggygria),* Butterbur Extract (*Petasites hybridus),* Goji Berry (*Lycium barbarum*), Milk Thistle Extract (*Silybum marianum*)*,* Honeysuckle (*Lonicera japonica*), Basalm of Peru (*Myroxylon pereirae),* Sage *(Salvia officinalis*), Cranberry Extract (*Vaccinium oxycoccos),* Amaranth Oil *(Amaranthus cruentus*), Pomegranate (*Punica granatum*), Yerbe Mate (*Ilex paraguariensis* Leaf Extract), White Lily Flower Extract (*Lilium candidum*), Olive Leaf Extract (*Olea europaea),* Phloretin (apple extract), Oat Flour (*Aveena sativa*), Lifenol (Hops: *Humulus lupulus)* Extract, Bugrane P (*Ononis spinoza*), Licochalcone (Licorice: *Glycyrrhiza inflate* extract ingredient), Symrelief (Bisabolol and Ginger extract), combinations of two or more thereof, and the like.

In one embodiment, the anti-inflammatory agent is a resorcinol. Particularly suitable substituted resorcinols include 4-hexyl resorcinol and 4-octylresorcinol, particularly 4-hexyl resorcinol. 4-Hexyl resorcinol is commercially available as "SYNOVEA HR" from Sytheon of Lincoln Park, NJ. 4-Octylresorcinol is commercially available from City Chemical LLC of West Haven, Connecticut.

By "extracts of feverfew," it is meant extracts of the plant *"Tanacetum parthenium,"* such as may be produced according to the details set for the in US Patent Application Publication No. 2007/0196523, entitled "PARTHENOLIDE FREE BIOACTIVE INGREDIENTS FROM FEVERFEW (TANACETUM PARTHENIUM) AND PROCESSES FOR THEIR PRODUCTION." One particularly suitable feverfew extract is commercially available as about 20% active feverfew, from Integrated Botanical Technologies of Ossining, NY.

In the skin care composition of the invention, the ratio of the amounts of the extract of *Malva neglecta* to the anti-inflammatory compound may be varied. For example, the extract and the anti-inflammatory compound may be present in a weight ratio (which is determined by dividing the amount by weight of the dry extract by the amount by weight of the anti-inflammatory compound) of about 0.001 to about 100, preferably about 0.01 to about 10, more preferably about 0.25 to about 2.

A variety of other materials may also be present in the compositions of the present invention. In certain preferred embodiments, the composition comprises one or more topical ingredients selected from the group consisting of: surfactants, chelating agents, emollients, humectants, conditioners, preservatives, opacifiers, fragrances and the like.

What is meant by an emollient is a compound that helps to maintain the soft, smooth, and pliable appearance of the skin *(e.g.,* by remaining on the skin surface or in the stratum corneum to act as a lubricant). Examples of suitable emollients include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY), and include, but are not limited to, petrolatum, hexyldecyl stearate and plant, nut, and vegetable oils such as macadamia nut oil, rice bran oil, grape seed oil, palm oil, prim rose oil, hydrogenates peanut oil, and avocado oil.

What is meant by a humectant is a compound intended to increase the water content of the top layers of skin (e.g., hygroscopic compounds). Examples of suitable humectants include those found Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY) and include, but are not limited to, glycerin, sorbitol or trehalose (e.g., α,α- trehalose, β,β-trehalose, α,β-trehalose) or a salt or ester thereof (e.g., trehalose 6-phosphate).

What is meant by a surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, Published in 2001 by Marcel Dekker, Inc New York, NY) and include, but are not limited to anionic surfactants such as sulfates, cationic surfactants such as betaines, amphoteric surfactants such as sodium coco glycinate, noionic surfactants such as alkyl polyglucosides.

Examples of suitable chelating agents include those which are capable of protecting and preserving the compositions of this invention. Preferably, the chelating agent is ethylenediamine tetracetic acid ("EDTA"), and more preferably is tetrasodium EDTA, available commercially from Dow Chemical Company of Midland, Michigan under the tradename, "Versene 100XL."

Suitable preservatives include, for example, parabens, quaternary ammonium species, phenoxyethanol, benzoates, DMDM hydantoin, organic acids and are present in the composition in an amount, based upon the total weight of the composition, from about 0 to about 1 percent or from about 0.05 percent to about 0.5 percent.

Any of a variety of conditioners which impart additional attributes, such as gloss to the hair, are suitable for use in this invention. Examples include, but are not limited to, volatile silicone conditioning agent having an atmospheric pressure boiling point less than about 220°C. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Michigan under the tradename, "DC-345" and mixtures thereof, and preferably include cyclomethicone fluids. Other suitable conditioners include cationic polymers, including polyquarterniums, cationic guar, and the like.

Any of a variety of commercially available pearlescent or opacifying agents are suitable for use in the composition. Examples of suitable pearlescent or opacifying agents include, but are not limited to, mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula: HO-(JO)ₐ-H, wherein J is an alkylene group having from about 2 to about 3 carbon atoms; and a is 2 or 3; fatty alcohols containing from about 16 to about 22 carbon atoms; fatty esters of the formula: KCOOCH₂L, wherein K and L independently contain from about 15 to about 21 carbon atoms; inorganic solids insoluble in the shampoo composition, and mixtures thereof

Any fragrance compositions suitable for use on skin may be used in accord with the present invention.

In certain preferred embodiments, the present invention is in the form of a substrate comprising a composition of the present invention. Any suitable substrate may be used. Examples of suitable substrates and substrate materials are disclosed, for example, in U.S. Published Application Nos. 2005/0226834 and 2009/0241242 which are incorporated herein by reference in their entirety.

In certain preferred embodiments, the substrate is a wipe, glove, or a facial mask. Preferably, such embodiments comprise a water-insoluble substrate as such is defined in the cited references above. For certain embodiments, the water-insoluble substrate may have a size and shape such that it covers the face of a human user to facilitate placing the water-insoluble substrate about the face of the user as a mask substrate. For example, the water-insoluble mask substrate may have openings for a mouth, nose, and/or eyes of the user. Alternatively, the water-insoluble substrate may have no such openings. Such a configuration without openings may be useful for embodiments of the invention in which the water-insoluble substrate is intended to be draped over a non-facial expanse of skin or if the water-insoluble substrate is intended to be used as wipe. The water-insoluble substrate may have various shapes, such as an angular shape *(e.g.,* rectangular) or an arcuate shape such as circular or oval. For certain embodiments, the substrate is a glove such as described in U.S. Published Application No 2006/0141014 which is incorporated herein in its entirety. In one embodiment of the invention, the product includes a plurality of water-insoluble substrates of different shapes.

The present invention further comprises a method of improving the barrier function and moisturization of skin by applying to skin in need of improving skin barrier function and moisturization an extract of *Malva neglecta,* in particular an extract of *Malva neglecta* aerial parts and/or roots. The method comprises for example topically applying a composition of the present invention comprising an extract of *Malva neglecta,* in particular an extract of *Malva neglecta* aerial parts and/or roots to skin in need of improving skin barrier function and moisturization. Such topical application may be to any skin in need of treatment on the body, for example skin of the face, lips, neck, chest, back, arms, axilla, hands, feet and/or legs. Preferably, the extract is a non-polar and / or lipophilic extract of *Malva neglecta.* The extract of *Malva neglecta* is preferably applied in an effective amount that results in the desired improvement of skin barrier function being achieved.

The present invention further comprises a method of improving the appearance of at least one sign of skin aging by applying to skin in need of improving the appearance of at least one sign of skin aging an extract of *Malva neglecta,* in particular an extract of *Malva neglecta* aerial parts and/or roots. The method comprises for example topically applying a composition of the present invention comprising an extract of *Malva neglecta,* in particular an extract of *Malva neglecta* aerial parts and/or roots to skin in need of treatment of at least one sign of skin aging. Such topical application may be to any skin in need of treatment on the body, for example skin of the face, lips, neck, chest, back, arms, axilla, hands, feet and/or legs. Preferably, the extract is a non-polar and / or lipophilic extract of *Malva neglecta.* The extract of *Malva neglecta* is preferably applied in an effective amount that results in the desired improvement in the appearance of at least one sign of skin aging being achieved.

The present invention further comprises a method of lightening the skin by applying to skin in need of skin lightening treatment an extract of *Malva neglecta,* in particular an extract of *Malva neglecta* aerial parts and/or roots. The method comprises for example topically applying a composition of the present invention comprising an extract of *Malva neglecta,* in particular an extract of *Malva neglecta* aerial parts and/or roots to skin in need of skin lightening treatment. Such topical application may be to any skin in need of treatment on the body, for example skin of the face, lips, neck, chest, back, arms, axilla, hands, feet and/or legs. Preferably, the extract is a non-polar and / or lipophilic extract of *Malva neglecta.* The extract of *Malva neglecta* is preferably applied in an effective amount that results in the desired skin lightening being achieved.

Any suitable method of applying the composition to the skin in need may be used. For example, the composition may be applied directly from a package to the skin in need, by hand to the skin in need, or may be transferred from a substrate such as a wipe or mask, or a combination of two or more thereof. In other embodiments, the composition may be applied via a dropper, tube, roller, spray, and patch or added to a bath or otherwise to water to be applied to the skin, and the like. The composition may be applied in a variety of manners /forms, including, without limitation, as a leave-on cream, mask, and / or serum.

In certain preferred embodiments, the methods of the present invention comprise applying at least two different compositions or products comprising a *Malva neglecta* extract to the skin. For example, the methods may comprise applying a first composition comprising *Malva neglecta* extract to skin in need of improving skin barrier efficacy and moisturization, followed by applying a second composition comprising *Malva neglecta* extract that is different from the first composition, to the skin in need of treatment. In certain preferred embodiments, the first and second composition may be independently selected from the group consisting of lotions, cleansers, masks, wipes, creams, serums, gels, and the like. In certain preferred embodiments, at least one of the first and second compositions is a cleanser, lotion, cream, essence, or serum and the other is a facial mask or wipe. In certain other preferred embodiments, at least one of the first and second compositions is a cleanser and the other is a lotion or cream.

### EXAMPLES

The following test methods were used in the Examples.

### Assay 1: PPARδ transactivation assay

Control samples of HEK293 transfected with human peroxisome proliferator-activated receptor delta (hPPARδ) ligand binding domain were prepared and harvested as indicated below, but without addition of any extract. Upon treatment, the transactivation of hPPARδ was measured. Cells were lysed and luminescence of the luciferase signal was measured. The potency of the extracts was determined by comparing the fold increase achieved by the extracts against the vehicle-treated control.

Specifically, plasmid containing human PPARδ ligand binding domain (LBD) fused with yeast Gal4 DNA binding domain, and Gal4-luciferase vectors were supplied by Janssen Research & Development, LLC. Human HEK239 cells were grown in DMEM+10%FBS+1% Glutamine+1% Na Pyruvate to about 70% preconfluency in T75 flasks. Cells were transiently transfected with two plasmids (1:1 ratio) using Lipofectamin 2000 reagent (Life technologies, Grand Island, NY) in T75 flask. The transfection protocol for a T75 flask included treating cells with 1) 10µg DNA (5µg of each vector) + 1.25mL OptiMEM; 2) 25 µl lipofectamine + 1.25mL OptiMEM; 3) incubating for 5 min; 4) mixing together; 5) incubating 20 min; and 6) adding to 10 mL growth media without P/S. After 20-24h transfection, media were removed and cells were lifted and counted. Compound treatment was prepared in phenol-red free growth media with 0.1% final DMSO concentration (vehicle) and then added into designated 96 well plates. 40,000 cells were added onto each well in additional 100µl of phenol-red free growth media. Final volume for each reaction was 200µl. Following 20-24h treatment, media were removed and kept for LDH assay. 25µl of 1xPLB lysis buffer was added in each well and incubated for 10 min with gentle shaking. 100µl of luciferase detection buffer (Promega luciferase assay system Cat# E1501) was added to measure luciferase activity.

### Assay 2: PPARα transactivation assay

hPPARα transactivation activity was measured by luciferase assay using hPPARα assay kit (Cat#IB00111) from INDIGO biosciences (State College, PA) and the manufacturer's instructions for the assay were followed. In brief, test materials were prepared at the appropriate dilution series of 2x-concentrated reference agonist (GW590735) and an appropriate dilution series of 2x-concentrated test material(s) to be assayed in compound screening media (supplied in the kit). 10mL of cell recovery media (supplied in the kit) was added to frozen cell pellet (hPPARα cells) and defrosted at a water bath. 100µl of hPPARα cells and prepared test materials were dispensed into each well of the 96 well assay plate (final volume was 200µl per well). Following an overnight incubation, the treatment media were discarded and 100µl of Luciferase Detection Reagent (LDR, supplied) was added per well. The intensity of light emission from each sample well was quantified using a plate-reading luminometer (SpectraMax).

### Assay 3: Gene expression

Samples were isolated from primary human keratinocytes and skin equivalents that had been treated with extracts dissolved in DMSO or DMSO without extracts (as control) for 24 hours using Qiagen RNeasy kit with DNase I digestion (Cat#79254) (Valencia, CA). Reverse transcription was performed using High Capacity cDNA kit (Life technologies Cat#4368814). 40 to 60ng of cDNA samples were used for QPCR reaction. Taqman gene expression assay was purchased from Life Technologies (Grand Island, NY). QPCR reaction was performed using ABI 7500 fast amplifier. The PCR primers used are presented in Table 1. All gene expression data were normalized by reference genes, polymerase (RNA) II polypeptide A (POLR2A) or/and ribosomal protein, large, PO (RPLPO). Relative gene expression was calculated by comparative CT method.

**Involucrin** is a protein of human epidermis encoded by the *IVL* gene and it contributes to the cell envelope formation that protects corneocytes in the skin.

**Transglutaminase** catalyzes the formation of bonds between a free amine group and the gamma-carboxamide group of glutamine that exhibit high resistance to proteolytic degradation and enhance the natural barrier of the skin.

**Sphingomyelin phosphodiesterase 3** is an enzyme that in humans is encoded by the *SMPD3* gene and is involved in ceramide synthesis.

**Aquaporin (AQP3)** is a water and glycerol channel, playing an essential role in skin hydration.

**HBEGF** is the predominant growth factor in the epithelialization required for cutaneous wound healing. The mitogenic and migratory effects of HB-EGF on keratinocytes and fibroblasts promote dermal repair and angiogenesis necessary for wound healing and is a major component of wound fluids. HB-EGF displays target cell specificity during the early stages of wound healing being released by macrophages, monocytes, and keratinoctyes. HB-EGF cell surface binding to heparan sulfate proteoglycans enhances mitogen promoting capabilities increasing the rate of skin wound healing, decreasing human skin graft healing times, and promotes rapid healing of ulcers, burns, and epidermal split thickness wounds.

**Table 1 - PCR primers**

| **Life Technologies (Applied biosystems)** | |
|---|---|
| **Gene Symbol** | **Catalog Number** |
| ANGPTL4 | Hs01101127_m1 |
| PLIN2 | Hs00912671-m1 |
| PPARδ | Hs04187066_g1 |
| PPARα | Hs00947539_m1 |
| IVL (Involucrin) | Hs00846307_s1 |
| TGM1 | Hs01070310_m1 |
| SMPD3 | Hs00920354_m1 |
| GBA | Hs00986836_g1 |
| SPTLC2 | Hs00191585_m1 |
| ABCA12 | Hs00917552_m1 |
| ELOVL4 | Hs00224122_m1 |
| UGCG | Hs00234293_m1 |
| CERS3 | Hs00698859_m1 |
| POLR2A | Hs00172187_m1 |
| CLDN7 | Hs00600772_m1 |
| AQP3 | Hs01105469_g1 |
| HBEGF | Hs00181813_m1 |

### Assay 4: Hyaluronic acid (HA) Secretion

Human dermal fibroblasts were maintained in flask in growth medium consisting of DMEM plus 10% fetal bovine serum, 50 units/ml penicillin and 50µg/ml streptomycin. Cells were seeded at 10,000 cells per well in a 96 well plate. After 24 hours incubation, cells were treated with test articles dissolved in DMSO or DMSO without extracts (as control) prepared in DMEM+2%FBS. Culture media was collected at 48 hours post-treatment, and measured for HA (Hyaluronic acid) secretion using Hyaluronan ELISA kit (Echelon, cat. #K-1200) following the manufacturer protocol. To assess activity, the colorimetric chance was measured at 405 nm and the results expressed as a fold change over untreated controls.

### Assay 5: Extra-cellular matrix gene expression

Changes in the transcription of extra-cellular matrix genes were measured by quantitative polymerase chain reaction (qPCR) assays. Dermal fibroblasts and epidermal skin equivalents were treated with extracts dissolved in DMSO or with DMSO alone (as control) in prepared media for 24 hours prior to mRNA extraction. The mRNA of primary human dermal fibroblasts and epidermal skin equivalents (MatTak, Epi-200) were isolated using the RNeasy Mini Kit (250), (Qiagen Catalog # 74106). The mRNA was reverse transcribed to complementary DNA (cDNA) using SuperScript III First Stand, (Invitrogen Catalog # 18080-400). The qPCR analysis was performed using Power SYBR Green PCR Master Mix, (Applied Biosystems Catalog #4367659), and run on a 7500 Real Time PCR system (Applied Biosystems) using the following conditions: 95 °C for 15 seconds, 60 °C for 1 minute with 40 cycles.

The primers of target genes are listed in Table 2 below. The potency of the test compounds was determined by comparing the fold change achieved by the test compounds against the control.

**Table 2 - Primers for PCR assays**

| **Gene** | **Gene Symbol** | **SABiosciences/ QIAGEN Catalog Number** |
|---|---|---|
| Collagen VII | COL7A1 | PPH01968A |
| Elastin | ELN | PPH06895F-200 |
| Hyaluronan synthase 3 | HAS3 | PPH10335E |

### Assay 6 - Determination of Ceramide Profile by High-Performance Thin-Layer Chromatography

### Sample Extraction and Condensation

Skin equivalents or 0.5-1x10⁶ cells were homogenized with 2mL chloroform:methanol (2:1) and transferred to a vial containing 1mL Phosphate-Buffered Saline Solution. Homogenizer was rinsed with 2 2mL portions of chloroform:methanol (2:1) and the rinses were added to the vial containing the extract and the PBS. The mixture was vortexed and the phases were allowed to separate. The organic phase was evaporated to dryness under vacuum. Sample residue dissolved in 200µL chloroform:methanol (2:1)

### High-Performance Thin-Layer Chromatography

The residue was dissolved in 200µL chloroform:methanol (2:1). Twenty microliters and 40uL of sample solution was applied on the HPTLC plate (Whatman Partisil) using CAMAG Automatic TLC Sampler 4 and separated using the following sequential development system:
(1) dichloromethane:ethyl acetate:acetone (80:16:4), (2) chloroform:methanol:acetone (76:16:8), and (3) hexane:chloroform:acetic acid:acetone:methanol (6:80:0.1:10:4). The plates were stained with 3% copper acetate in 8% phosphoric acid and charred at 160°C.

### Quantification

Samples were applied in parallel for positional corrections and compared to a similarly prepared blank extract (tape strip without exposure to skin lipids). Quantification was performed against known quantities of Ceramide III standard (Cosmoferm) by densitometry (CAMAG).

### Assay 7 - Pigmentation assays

Melanin assays in B16-F10 cell line: Murine B16-F10 cells were seeded in 24 well plate and allowed to adhere overnight. Cells were then exposed to 20mJ of UVB from a solar simulator (Oriel instruments). After SSR exposure, the cells were treated with different concentration of the extract dissolved in 0.1% DMSO. DMSO (0.1%) was also included as a vehicle control. The cells were extracted after 48 hours of treatment, lysed in RIPA buffer, and collected in 1.5ml test tube. The extraction was centrifuged at 14000 rpm for 10 mins. The cell pellets were dissolved in 1N NaOH, incubated at 60 °C for 1hr and used to calculate melanin concentration, measured spectrophotometrically (Versa max, Molecular devices) at 470nm. Melanin concentration was normalized to protein concentration by bicinchonic acid (BCA) assay.

Melanin assays in 3D skin equivalent model: Pigmented epidermal equivalents (MelanoDerm™) from MatTek Corporation (Ashland, MA), with melanocytes derived from Black donor, were maintained EPI-100-LLMM media according to manufacturer's protocol. The equivalents were topically treated with vehicle (70% ethanol/30% propelyene glycol) or 2.5%, 1.25% & 0.625% of *Malva Neglecta* extract once daily for 5 days, in duplicate. On day 9, the tissues were evaluated by measurement of skin luminosity (L-value) was performed using a spectrophotometer (Konica Minolta CM-2600d) & 3).

The following examples illustrate the preparation and efficacy of *Malva neglecta* extracts.

### Example 1: Preparation of Malva neglecta extract from aerial parts (E1)

*Malva neglecta* plants were wild-collected in New York. Species identification was based on gross morphological characteristics [Gleason & Cronquist, Manual of Vascular Plants; D Van Nostrand Company, NY: p. 462-463]. Plants were cleaned of soil and debris and separated into aerial parts and roots. Approximately 80 g of fresh aerial plant material was homogenized in a blender with 200 mL of 80% aqueous methanol; the suspension was maintained in constant motion for 24 hours. The resulting suspension was then filtered and dried under low pressure using a rotary evaporator not exceeding 40 °C. After filtration, the left over raw material was again extracted as described above. The combined dry mass from both extractions was designated the crude extract, approximately 2.1 g, for a yield of 6.6%. The crude extract was resuspended in 100 mL water and subjected to liquid-liquid solvent partitioning in a separatory funnel using three equal parts hexane. The three hexane partitions were combined and dried under low pressure using a rotary evaporator not exceeding 40 °C to achieve a total mass of approximately 200 mg (E1), for a yield of 0.6%.

### Example 2: Preparation of Malva neglecta extracts from aerial parts (E2 and E3)

*Malva neglecta* plant was collected and extracted as described in Example 1 to get the crude extract. The crude extract was resuspended in 100 mL water and subjected to liquid-liquid solvent partitioning in a separatory funnel using three equal parts hexane followed by three equal parts ethyl acetate.

The three ethyl acetate partitions were combined and dried under low pressure using a rotary evaporator not exceeding 40 °C to achieve a total mass of approximately 73 mg (E2), for a yield of 0.2%. The remaining water phase was dried under low pressure using a rotary evaporator not exceeding 40 °C to achieve a total mass of approximately 1.8 g (E3), for a yield of 5.6%.

### Example 3: Preparation of Malva neglecta extract from roots (E4)

*Malva neglecta* plants were wild-collected in New York. Species identification was based on gross morphological characteristics [Gleason & Cronquist, Manual of Vascular Plants; D Van Nostrand Company, NY: p. 462-463]. Plants were cleaned of soil and debris and separated into aerial parts and roots. Approximately 30 g of fresh root material was homogenized in a blender with 100 mL of 80% aqueous methanol; the suspension was maintained in constant motion for 24 hours. The resulting suspension was then filtered and dried under low pressure using a rotary evaporator not exceeding 40 °C. The plant material remaining was resuspended in 100mL of 80% aqueous methanol and maintained in constant motion a second time. After 24 hours, the suspension was filtered and dried under low pressure using a rotary evaporator not exceeding 40 °C. The combined dry mass from both extractions was designated the crude extract, approximately 3.3 g, for a yield of 4.1%. The crude extract was resuspended in 100 mL water and subjected to liquid-liquid solvent partitioning in a separatory funnel using three equal parts hexane. The three hexane partitions were combined and dried under low pressure using a rotary evaporator not exceeding 40 °C to achieve a total mass of approximately 200 mg (E4), for a yield of 0.3%.

### Example 4: Preparation of Malva neglecta extracts from roots (E5 and E6)

*Malva neglecta* plant was collected and extracted as described in Example 3 to get the crude extract. The crude extract was resuspended in 100 mL water and subjected to liquid-liquid solvent partitioning in a separatory funnel using three equal parts hexane followed by three equal parts ethyl acetate.

The three ethyl acetate partitions were combined and dried under low pressure using a rotary evaporator not exceeding 40 °C to achieve a total mass of approximately 200 mg (E5), for a yield of 0.3%. The remaining water phase was dried under low pressure using a rotary evaporator not exceeding 40 °C to achieve a total mass of approximately 3.0 g (E6), for a yield of 3.8%.

### Example 5: Preparation of Malva moschata extract from aerial parts (E7)

*Malva moschata* plants were wild-collected in New Jersey. Species identification was based on gross morphological characteristics [Gleason & Cronquist, Manual of Vascular Plants; D Van Nostrand Company, NY: p. 462-463]. Approximately 100 g of fresh whole plant material was homogenized in a blender with 200 mL of 80% aqueous methanol; the suspension was maintained in constant motion for 24 hours. The resulting suspension was then filtered and dried under low pressure using a rotary evaporator not exceeding 40 °C. The plant material remaining was resuspended in 200mL of 80% aqueous methanol and maintained in constant motion. After 24 hours, the suspension was filtered and dried under low pressure using a rotary evaporator not exceeding 40 °C. The combined dry mass from both extractions was designated the crude extract, approximately 5 g, for a yield of 5%. The crude extract was resuspended in 100 mL water and subjected to liquid-liquid solvent partitioning in a separatory funnel using three equal parts hexane. The three hexane partitions were combined and dried under low pressure using a rotary evaporator not exceeding 40 °C to achieve a total mass of approximately 714 mg (E7), for a yield of 0.7%.

### Example 6: Preparation of Malva moschata extract from aerial parts (E8 and E9)

*Malva moschata* plant was collected and extracted as described in Example 5 to get the crude extract.

The crude extract was resuspended in 100 mL water and subjected to liquid-liquid solvent partitioning in a separatory funnel using three equal parts hexane, followed by three equal parts ethyl acetate.

The three ethyl acetate partitions were combined and dried under low pressure using a rotary evaporator not exceeding 40 °C to achieve a total mass of approximately 254 mg (E8), for a yield of 0.3%. The remaining water phase was dried under low pressure using a rotary evaporator not exceeding 40 °C to achieve a total mass of approximately 4 g (E9), for a yield of 4%.

### Example 7:

Samples of *Malva neglecta* extracts E1 to E6 were compared for transactivation of hPPARδ using the method of Assay 1.

The results are shown in Table 3.

**Table 3: PPARδ activation over vehicle control**

| **Sample ID** | **plant part** | **extraction media** | **concentration** | **PPARδ Transactivation Assay** | |
|---|---|---|---|---|---|
| | | | | **fold induction** | **Stdev** |
| E1 | Aerial | HEXANE | 1 ppm | 1.51 | 0.11 |
| E1 | | | 5 ppm | 1.86 | 0.10 |
| E1 | | | 25 ppm | 4.41 | 0.21 |
| E1 | | | 50 ppm | 5.13 | 0.59 |
| E4 | Root | HEXANE | 1 ppm | 1.54 | 0.15 |
| E4 | | | 5 ppm | 1.63 | 0.05 |
| E4 | | | 25 ppm | 3.44 | 0.16 |
| E4 | | | 50 ppm | 4.07 | 0.04 |
| E2 | Aerial | Ethyl acetate | 1 ppm | 1.13 | 0.24 |
| E2 | | | 5 ppm | 1.16 | 0.15 |
| E2 | | | 25 ppm | 1.82 | 0.11 |
| E2 | | | 50 ppm | 2.11 | 0.11 |
| E6 | Root | Water | 1 ppm | 1.34 | 0.05 |
| E6 | | | 5 ppm | 1.05 | 0.32 |
| E6 | | | 25 ppm | 1.35 | 0.04 |
| E6 | | | 50 ppm | 1.45 | 0.09 |
| E3 | Aerial | Water | 1 ppm | 1.30 | 0.11 |
| E3 | | | 5 ppm | 1.28 | 0.03 |
| E3 | | | 25 ppm | 1.29 | 0.11 |
| E3 | | | 50 ppm | 1.33 | 0.06 |
| E5 | Root | Ethyl acetate | 1 ppm | 0.97 | 0.06 |
| E5 | | | 5 ppm | 0.98 | 0.04 |
| E5 | | | 25 ppm | 1.04 | 0.09 |
| E5 | | | 50 ppm | 1.07 | 0.09 |

By activating PPAR *Malva neglecta* extracts would be expected to increase expression of proteins which help strengthen the barrier of skin.

### Example 8:

Samples of *Malva neglecta* extracts with significant PPARδ activation over control (E1, E2 and E4) were compared for transactivation of PPARα using the method of Assay 2. The results are shown in Table 4.

All extracts with significant PPARδ activation has also exhibited significant PPARα activation. Moreover, E1 has showed the highest activation of both forms of PPAR receptor.

The results are shown in Table 4.

**Table 4: PPARα activation over vehicle control**

| **Sample tested** | **plant part** | **fraction** | **concentration** | **PPARα Transactivation Assay** | |
|---|---|---|---|---|---|
| | | | | **fold induction** | **Stdev** |
| E1 | Aerial | HEXANE | 1 ppm | 1.33 | 0.12 |
| E1 | | | 5 ppm | 2.47 | 0.04 |
| E1 | | | 25 ppm | 3.78 | 0.03 |
| E1 | | | 50 ppm | 2.19 | 0.30 |
| E4 | Root | HEXANE | 1 ppm | 0.86 | 0.10 |
| E4 | | | 5 ppm | 1.36 | 0.15 |
| E4 | | | 25 ppm | 2.47 | 0.26 |
| E4 | | | 50 ppm | 1.87 | 0.15 |
| E2 | Aerial | Ethyl acetate | 1 ppm | 1.06 | 0.08 |
| E2 | | | 5 ppm | 1.60 | 0.01 |
| E2 | | | 25 ppm | 2.71 | 0.11 |
| E2 | | | 50 ppm | 2.80 | 0.44 |

### Example 9: Transcription of ceramide synthesis genes, differentiation markers, and PPAR target genes

Extracts E1 and E7 prepared similarly from two different species of *Malva* (*Malva neglecta* and *Malva moschata,* respectively) were tested for increase in ceramide synthesis gene transcription, differentiation markers and PPAR target genes in accord with the method of Assay 3 described above and the results are given in Tables 5-8 below.

**Table 5: Results of PCR experiments using human keratinocyte cell culture showing results for PPAR PPARα/δ and PPAR target genes.**

| **Test article** | **Concentration (µg/mL)** | **ANGPTL4** | **PLIN2** | **PPARδ** | **PPARα** |
|---|---|---|---|---|---|
| | | **Fold Change** | **Fold Change** | **Fold Change** | **Fold Change** |
| Control | VEH(0.5% DMSO) | 1.0 | 1.0 | 1.0 | 1.0 |
| E1 | 25 | 7.9 | 13.3 | 6.5 | 2.3 |
| E7 | 25 | 3.4 | 4.5 | 2.2 | 2.1 |

**Table 6: Results of PCR experiments using human keratinocyte cell culture showing results for cellular differentiation markers.**

| **Test article** | **Concentration (µg/mL)** | **INV** | **TGM1** |
|---|---|---|---|
| | | **Fold Change** | **Fold Change** |
| Control | VEH(0.5% DMSO) | 1.0 | 1.0 |
| E1 | 25 | 4.1 | 2.2 |
| E7 | 25 | 3.1 | 1.6 |

**Table 7: Results of PCR experiments using human keratinocyte cell culture showing results for ceramide synthesis and transport genes.**

| **Test article** | **Concent ration (µg/mL)** | **SMPD3** | **GBA** | **SPTLC2** | **ABCA12** | **ELOVL4** | **UGCG** | **CERS3** |
|---|---|---|---|---|---|---|---|---|
| | | **Fold Change** | **Fold Change** | **Fold Change** | **Fold Change** | **Fold Change** | **Fold Change** | **Fold Change** |
| Control | VEH(0.5 % DMSO) | 1.0 | 1.0 | 1.0 | 1.0 | 1.0 | 1.0 | 1.0 |
| E1 | 25 | 9.1 | 3.1 | 1.3 | 4.2 | 5.7 | 7.0 | 3.9 |
| E7 | 25 | 5.1 | 1.9 | 1.3 | 1.7 | 3.3 | 2.8 | 1.6 |

**Table 8: Results of PCR experiments using epidermal skin equivalents**

| **Extract** | **Concentra -tion (mg/mL)** | **ANGPTL4** | **UGCG** | **Involucrin** | **SMPD3** | **CLDN7** | **AQP3** | **HBEGF** |
|---|---|---|---|---|---|---|---|---|
| | | **Fold Change** | **Fold Change** | **Fold Change** | **Fold Change** | **Fold Change** | **Fold Change** | **Fold Change** |
| Vehicle | 0.1% DMSO | 1 | 1 | 1 | 1 | 1 | 1 | 1 |
| E1 | 50 | 3.6 | 1.9 | 2.2 | 2.4 | 3.9 | 9.3 | 13.8 |
| E7 | 50 | 1.8 | 1.5 | 1.7 | 1.9 | 1.7 | 3.5 | 6.5 |

All data from Table 5-8 for E1 indicating mostly superior gene expressions to gene expressions for E7 implying superior efficacy of E1 (*Malva neglecta* extract) over E7 (*Malva moschata* extract).

By increasing expression of genes involved in producing skin lipids and enhancing the differentiation of skin, *Malva neglecta* extracts would be expected to help strengthen the barrier of skin.

### Example 10: Determination of Ceramides in human primary keratinocytes

Extract E1 was tested for ceramide levels using the method of Assay 6 described above. The results are given in Table 9 below.

**Table 9: Results of ceramide production in a keratinocyte cell culture model**

| **Extract** | **Concentration (µg/mL)** | **Percent of control** |
|---|---|---|
| Vehicle | 0.1% DMSO | 100 |
| E1 | 25 | 124 |

The preceding examples 9 and 10 demonstrate the ability of non-polar extracts of *Malva neglecta* (E1) to induce expression of ceramide synthesis and transport genes, as well as functionally to increase the endogenous production of ceramides. In addition, there was an increase in transcription of skin differentiation related genes. Ceramides are lipid components of the skin which are an important part of the outer layer of skin, and therefore important in protecting the barrier function of skin. Collectively, these changes indicate that non-polar extract of *Malva neglecta* (E1) has the ability to induce physiological changes that positively affect skin barrier function and improve the moisturization and appearance of dry skin including reducing the appearance of skin flakes. Non-polar extracts of *Malva moschata* (E7) did not show the same degree of gene expression increases, demonstrating that these effects are superior in the case of *Malva neglecta.*

By increasing the production of ceramides, *Malva neglecta* extracts would be expected to increase the help strengthen the barrier of skin.

### Example 11: Transcription of extra-cellular matrix genes

Extracts E1 - E9 were tested for changes in transcription of extra-cellular matrix genes in accord with the method of Assay 5 above. The results are given in the Tables 10 and 11 below.

**Table 10: Results from PCR analysis of Human primary keratinocyte cell culture**

| **Test article** | **Concentration (µg/mL)** | **Collagen 7** | **HAS3** |
|---|---|---|---|
| | | **Fold Change** | **Fold Change** |
| Vehicle Control | 0.5% DMSO | 1 | 1 |
| E1 | 5 | 1.5 | 1.715 |
| E1 | 25 | 2.63 | 4.035 |
| E7 | 5 | 1.42 | 1.065 |
| E7 | 25 | 1.525 | 3.655 |

**Table 11: Results from PCR analysis of epidermal skin equivalents**

| **Test article** | **Concentration** | **Col7A1** | **Elastin** | **HAS3** |
|---|---|---|---|---|
| | | **Fold Change** | **Fold Change** | **Fold Change** |
| Vehicle Control | untreated | 1.00 | 1.00 | 1.00 |
| E7 | 5% | 0.96 | 0.21 | 1.13 |
| E8 | 5% | 1.53 | 0.95 | 1.07 |
| E9 | 5% | 0.39 | 0.48 | 0.53 |
| E4 | 5% | 7.86 | 39.27 | 12.75 |
| E5 | 5% | 1.34 | 1.83 | 1.28 |
| E6 | 5% | 0.86 | 2.37 | 1.20 |
| E3 | 5% | 0.53 | 1.06 | 0.80 |
| E2 | 5% | 1.00 | 0.25 | 0.67 |
| E1 | 5% | 3.66 | 15.74 | 7.06 |

### Example 12: Hyaluronic acid (HA) Secretion

Extracts E1 was tested for hyaluronic acid secretion using the method of Assay 4 described above. The results are given in Table 12 below.

**Table 12: Increase in hyaluronic acid secretion**

| **Test article** | **Concentration (µg/mL)** | **HA secretion** |
|---|---|---|
| | | **Fold Change** |
| Vehicle control (DMSO 0.05%) | - | 1 |
| E1 | 5 | 1.5 |
| E1 | 10 | 1.43 |
| E1 | 25 | 1.66 |
| TGF-b, 20ng/ml (positive control) | | 2.54 |

The preceding examples 11 and 12 demonstrate the ability of *Malva neglecta* extracts (E1 and E4) to induce expression of extracellular matrix genes. None of the extracts of *Malva moschata* (E7-E9) that were tested showed any significant induction of the same genes. These results demonstrate an ability of *Malva neglecta,* but not *Malva moschata,* to induce superior biological benefits which would be expected to improve the appearance of skin wrinkles, fine lines, sagging or lax skin and aged skin. In particular, collagen, elastin, and hyaluronic acid are important components of the skin extra-cellular matrix, giving the skin elasticity and strength. The amount of these molecules in skin declines with age, and an increase would be expected to improve the appearance of skin wrinkles, fine lines, sagging or lax skin and aged skin. The *Malva neglecta* extract (E1 and E4) enhanced the expression of extracellular matrix genes linked to the production of these molecules, and the HA ELISA data showed that *Malva neglecta* extract (E1) enhanced the HA secretion in human dermal fibroblast cell culture.

By increasing expression of extracellular matrix genes in skin genes, *Malva neglecta* extracts would be expected to help strength the support function of skin thereby improving the appearance of one or more signs of aging in skin.

### Example 13: Melanogenesis inhibition with Malva neglecta extract (E1)

Extract E1 was tested for changes in Melanin production (melanogenesis) by using the method of Assay 7 described above. The results are given in Tables 13 and 14 below.

**Table 13: Results of melanin production in B16 cell line in B16 cell line**

| **Treatment** | **Concentration** | **OD/protein** | **% reduction as of control** |
|---|---|---|---|
| Untreated | vehicle | 3.55 | 0 |
| E1 | 25ug/ml | 1.69 | 47.68 |
| E1 | 10ug/ml | 2.66 | 63.77 |
| E1 | 5ug/ml | 2.76 | 77.91 |
| E1 | 1ug/ml | 3.28 | 92.43 |

Treatment with *Malva neglecta* extract (E1) decreased melanin production in the murine B16 melanocyte cell model, indicating the extract may be used to impart even tone and inhibit pigmentation to the skin in need of lightening.

**Table 14: Results of melanin production in 3D skin equivalent model**

| **Treatment** | **Concentration** | **L (measure of skin lightness)** |
|---|---|---|
| **70%ethanol/30%propelyene glycol** | **Vehicle** | **39.79** |
| **E1** | **0.625%** | **40.23** |
| **E2** | **1.25%** | **41.17** |
| **E3** | **2.5%** | **42.66** |

Treatment of 3D skin equivalent model with *Malva neglecta* extract increased L values. L values are directly proportion to the degree of skin lightness as described elsewhere in the specification. Higher L values after treatment with E1 indicated the extract may be used to impart even tone and inhibit skin pigmentation.

### Example 14: Compositions containing Malva neglecta extract

Example of four skin care compositions according to the invention are presented below in Tables 15-18 along with their methods of preparation

**Table 15**

| **Trade Name** | **INCI Name** | **% weight** |
|---|---|---|
| Deionized Water | Water | 70.64 |
| Sodium Chloride | Sodium Chloride | 0.01 |
| *Malva neglecta* herb Extract | *Malva neglecta* extract | 1.00 |
| Snow White Petrolatum | Petrolatum | 4.00 |
| ISOFOL 28 | Dodecylhexadecanol | 2.50 |
| DOW CORNING Q7-9120 (20 CS) | Dimethicone | 1.25 |
| KESSCO IPP | Isopropyl Palmitate | 3.00 |
| VARISOFT TA-100 | Distearyldimonium Chloride | 5.00 |
| Glycerin | Glycerin | 12.00 |
| Benzyl Alcohol | Benzyl Alcohol | 0.60 |

The composition shown in Table 15 above can be prepared as follows: water is added to a process vessel. Mixing is begun and salt is added and mixed until dissolved. Heat is applied and mixing continued until 85°C. is reached. Glycerin is then added while mixing continued while temperature is maintained at 85°C. Varisoft TA 100 is added, as is petrolatum and Isofol 28, DC Q7-9120 20 cs., and isopropyl palmitate. The composition is mixed at 85°C for another 10-15 minutes. The composition is then removed from heat and continued to mix and cooled. At 40°C, benzyl alcohol is added, *Malva neglecta* herb extract is weighed and dissolved in Glycerin and added to the mixture,, q.s. with water and continued to be mixed and cooled to 30-35°C. The composition is then filled into packaging.

**Table 16: Second example of skin care composition**

| **Trade Name** | **INCI Name** | **% weight** |
|---|---|---|
| Deionized Water | Water | 65.55 |
| Snow White Petrolatum | Petrolatum | 4.00 |
| ISOFOL 28 | Dodecylhexadecanol | 2.50 |
| DOW CORNING Q7-9120 (20 CS) | Dimethicone | 1.25 |
| BHT | BHT | 0.10 |
| KESSCO IPP | Isopropyl Palmitate | 3.00 |
| VARISOFT TA-100 | Distearyldimonium Chloride | 5.00 |
| *Malva neglecta* herb Extract | *Malva neglecta* extract | 5.00 |
| Glycerin | Glycerin | 12.00 |
| Retinol 10S | *Glycine Soja* (Soybean) Oil and Retinol | 1.00 |
| Benzyl Alcohol | Benzyl Alcohol | 0.60 |

The composition shown in Table 16 above can be prepared as follows: Water is added to a process vessel and the temperature is set to 85 °C. Mixing is begun and glycerin is added and mixed until dissolved. Varisoft TA-100 and Petrolatum are added and Isofol 28, DC Q7-9120 20 cs., and isopropyl palmitate. The composition is mixed at 85°C for another 10-15 minutes. The composition is then removed from heat and Retinol 10S and *MALVA NEGLECTA* herb extract are added to the mix and cooled. At 40°C, benzyl alcohol is added, q.s. with water and continued to be mixed and cooled to 30-35°C. The composition is then filled into packaging.

**Table 17: Third example of skin care composition**

| **Trade Name** | **INCI Name** | **% weight** |
|---|---|---|
| Purified water | Deionized Water | 77.90 |
| HYDROLITE 5 | Pentylene glycol | 5.00 |
| *Malva neglecta* herb Extract | *Malva neglecta* extract | 0.1 |
| NATRULON OSF | Carthamus Tinctorius Oleosome | 10.00 |
| FINSOLV TN | C₁₂-₁₅ Alkyl Benzoate | 4.00 |
| ARISTOFLEX AVC | Ammonium Acryloyldimethyl-taurate/VP Copolymer | 2.00 |
| *Tanacetum parthenium* extract | *Chrysanthemum Parthenium* (Feverfew) Leaf/Flower/Stem Juice | 1.00 |

The composition shown in Table 17 above can be prepared as follows: *MALVA NEGLECTA* herb extract is weighed and dissolved in HYDROLITE 5 and deionized water is added to form Phase A. Oleosomes and Finsolv TN are mixed to form Phase B. Phase B is added to Phase A very slowly under continuous mixing. Mixing is continued for 15 minutes until a uniform emulsion is formed. ARISTOFLEX is added to the emulsion under continuous mixing at high speed to obtain a thick, smooth and homogenous formulation.

An inventive composition can be prepared by blending the ingredients according to the materials and amounts listed in Table 18.

**Table 18: Fourth example of skin care composition**

| **Trade Name** | **INCI Name** | **% weight** |
|---|---|---|
| Purified water | Water | 66.95 |
| Carbomer | Cross-linked polyacrylic acid | 0.60 |
| VERSENE NA | Disodium EDTA | 0.20 |
| Brij 72 | Steareth-2 | 0.75 |
| Brij 721 | Steareth-21 | 1.50 |
| FINSOLV TN | C₁₂-₁₅ Alkyl Benzoate | 2.00 |
| Dimethicone | Dow Corning Q7-9120 Silicone Fluid (20 cst) | 5.00 |
| Phenonip XB | Phenonip XB | 1.00 |
| LYS' LASTINE | *Peucedanum graveolens* (10% active) | 10.00 |
| SYMMATRIX | Maltodextrin, *Rubus Fruticosus* (Blackberry) Leaf Extract (10% active) | 10.00 |

| *Malva neglecta* herb Extract | *Malva neglecta* extract | 1.00 |
|---|---|---|
| Glycerin | Glycerin | 1.00 |

The composition shown in Table 18 above can be prepared as follows: an oil phase is prepared by adding C 12-15 alkyl benzoate to a clean glass beaker. Agitation is begun and the vessel is heated to 55-60° C. When the oil phase reaches 55°C or higher, Brij 72 and Brij 721 are added. When the oil phase reaches 55-60° C., it is held at that temperature and mixed for 15 min (or until uniform). The temperature is then held at 55-60° C. with mixing until addition to water phase. A water phase is prepared by adding water to a clean glass beaker. Agitation is begun and the vessel heated to 55-60° C. Disodium EDTA and Ultrez 10 are added. At 55-60° C., the ingredients are mixed for 15 min or until homogeneous. The temperature is then held at 55-60° C. with mixing for phasing. The oil phase is added to the water phase with increased agitation and then mixed at high speed for 10-20 min. At 50° C. or lower, dimethicone is added. At 40° C, or lower, Phenonip XB is added. The phases are then mixed for 10 min or until uniform. Sodium hydroxide is added (target pH is 5.4). The composition is then mixed for 10 min or until uniform. Lys'Lastine and SymMatrix are then added. *Malva neglecta* herb extract is weighed and dissolved in Glycerin and added to the mixture. This is mixed until uniform. Water is then added to QS and the composition is then mixed for 10 minutes.

### Aspects:

Aspect 1. A composition of a non-polar, or lipophilic, or non-polar lipophilic extract of *Malva neglecta,* a cosmetically acceptable topical carrier, and an active agent for improving skin barrier function.
Aspect 2. A composition as in aspect 1, wherein said composition is substantially free of plant biomass.
Aspect 3. A composition as in aspect 1, wherein said topical composition comprises from about 0.001% to about 90% of an extract of *Malva neglecta.*
Aspect 4. A composition as in aspect 1, wherein said topical composition comprises from about 0.01% to about 20% by weight of said extract of *Malva neglecta* herb.
Aspect 5. A composition as in aspect 1, wherein said topical composition comprises about 0.01 to about 5% of said extract of *Malva neglecta.*
Aspect 6. A composition as in aspect 1, wherein said composition comprises from about 0.01 % to about 2% of said extract of *Malva neglecta.*
Aspect 7. A composition of a non-polar, or lipophilic, or non-polar lipophilic extract of *Malva neglecta,* a cosmetically acceptable topical carrier, and an active agent for improving the appearance of at least one sign of aging in skin.
Aspect 8. A composition as in aspect 7, wherein said composition is substantially free of plant biomass.
Aspect 9. A compositionas in aspect 7, wherein said topical composition comprises from about 0.001% to about 90% of an extract of *Malva neglecta.*
Aspect 10. A composition as in aspect 7, wherein said topical composition comprises from about 0.01% to about 20% by weight of said extract of *Malva neglecta* herb.
Aspect 11. A composition as in aspect 7, wherein said topical composition comprises about 0.01 to about 5% of said extract of *Malva neglecta.*
Aspect 12. A composition as in aspect 7, wherein said composition comprises from about 0.01 % to about 2% of said extract of *Malva neglecta.*
Aspect 13. A composition of a non-polar, or lipophilic, or non-polar lipophilic extract of *Malva neglecta,* a cosmetically acceptable topical carrier, and an active agent for lightening skin.
Aspect 14. A composition as in aspect 13, wherein said composition is substantially free of plant biomass.
Aspect 15. A composition as in aspect 13, wherein said topical composition comprises from about 0.001% to about 90% of an extract of *Malva neglecta.*
Aspect 16. A composition as in aspect 13, wherein said topical composition comprises from about 0.01% to about 20% by weight of said extract of *Malva neglecta* herb.
Aspect 17. A composition as in aspect 13, wherein said topical composition comprises about 0.01 to about 5% of said extract of *Malva neglecta.*
Aspect 18. A composition as in aspect 13, wherein said composition comprises from about 0.01% to about 2% of said extract of *Malva neglecta.*
Aspect 19. A method of improving the barrier function of skin, said method comprising applying a topical composition of a non-polar and / or lipophilic extract of *Malva neglecta* to skin in need of improving skin barrier function to result in improved skin barrier function.
Aspect 20. A method as in aspect 19, wherein said extract of *Malva neglecta* is extracted using a solvent selected from the group consisting of liquid carbon dioxide with or without modifiers, aqueous ethanol, C₁-C₈ alcohols, C₁-C₈ alkanes, C₂-C₈ glycols _{/} polyols, C₅-C₈ cycloalkanes, C₁-C₈ alkyl ethers, C₁-C₈ aliphatics, ketones, methylene chloride, ethyl acetate, xylene, toluene, vegetable oil, mineral oil, and combinations thereof.
Aspect 21. A method as in aspect 20, wherein said solvent is selected from the group consisting ofhexanes, aqueous ethanol, ethanol, methanol, liquid carbon dioxide and combinations thereof.
Aspect 22. A method as in aspect 19, wherein said extract of *Malva neglecta* is extracted using a solvent system comprised of one solvent or combination of solvents having a dielectric constant of a value between about 1.5 and a value of about 40 at 20°C.
Aspect 23. A method as in aspect 22, wherein said solvent is selected from the group consisting of: pentanes, hexanes, heptanes, chloroform, methanol, ethanol, propanols, butanols, glycerine, butylenes glycol, propylene glycol, and liquid carbon dioxide with appropriate polarity modifier, and combinations thereof.
Aspect 24. A method as in aspect 19, wherein said extract is of the whole herb of *Malva neglecta.*
Aspect 25. A method as in aspect 19, wherein said topical composition comprises from about 0.001% to about 90% of an extract of *Malva neglecta.*
Aspect 26. A method as in aspect 19, wherein said topical composition comprises from about 0.01% to about 20% by weight of said extract of *Malva neglecta.*
Aspect 27. A method as in aspect 19, wherein said topical composition comprises about 0.01 % to about 5% of said extract of *Malva neglecta.*
Aspect 28. A method as in aspect 19, wherein said composition comprises from about 0.01% to about 2% of said extract of *Malva neglecta.*
Aspect 29. A method as in aspect 19, wherein said topical composition further comprises a cosmetically acceptable topical carrier.
Aspect 30. A method as in aspect 29, wherein said cosmetically acceptable topical carrier comprises a topical ingredient selected from the group consisting of surfactants, chelating agents, emollients, humectants, conditioners, preservatives, opacifiers, fragrances, and combinations thereof.
Aspect 31. A method as in aspect 19, wherein said topical composition comprises additional skin barrier improving agents.
Aspect 32. A method as in aspect 19, wherein said topical composition is substantially free of plant biomass.
Aspect 33. A method of improving the appearance of at least one sign of skin aging, said method comprising applying a topical composition of a non-polar and / or lipophilic extract of *Malva neglecta* to skin in need of improving the appearance of at least one sign of aging in skin therein to result in improvement of the appearance of at least one sign of aging in the skin.
Aspect 34. A method as in aspect 33, wherein said extract of *Malva neglecta* is extracted using a solvent selected from the group consisting of liquid carbon dioxide with or without modifiers, aqueousethanol, C₁-C₈ alcohols, C₁-C₈ alkanes, C₂-C₈ glycols / polyols, C₅-C₈ cycloalkanes, C₁-C₈ alkyl ethers, C₁-C₈ aliphatics, ketones, methylene chloride, ethyl acetate, xylene, toluene, vegetable oil, mineral oil, and combinations thereof.
Aspect 35. A method as in aspect 34, wherein said solvent is selected from the group consisting ofhexanes, aqueous ethanol, ethanol, methanol, liquid carbon dioxide and combinations thereof.
Aspect 36. A method as in aspect 33, wherein said extract of *Malva neglecta* is extracted using a solvent system comprised of one solvent or combination of solvents having a dielectric constant of a value between about 1.5 and a value of about 40 at 20°C.
Aspect 37. A method as in aspect 36, wherein said solvent is selected from the group consisting of: pentanes, hexanes, heptanes, chloroform, methanol, ethanol, propanols, butanols, glycerine, butylenes glycol, propylene glycol, and liquid carbon dioxide with appropriate polarity modifier, and combinations thereof.
Aspect 38. A method as in aspect 33, wherein said extract is of the whole herb of *Malva neglecta.*
Aspect 39. A method as in aspect 33, wherein said topical composition comprises from about 0.001% to about 90% of an extract of *Malva neglecta.*
Aspect 40. A method as in aspect 33, wherein said topical composition comprises from about 0.01% to about 20% by weight of said extract of *Malva neglecta.*
Aspect 41. A method as in aspect 33, wherein said topical composition comprises about 0.01 to about 5% of said extract of *Malva neglecta.*
Aspect 42. A method as in aspect 33, wherein said topical composition comprises from about 0.01% to about 2% of said extract of *Malva neglecta.*
Aspect 43. A method as in aspect 33, wherein said topical composition further comprises a cosmetically acceptable topical carrier.
Aspect 44. A method as in aspect 43, wherein said cosmetically acceptable topical carrier comprises a topical ingredient selected from the group consisting of surfactants, chelating agents, emollients, humectants, conditioners, preservatives, opacifiers, fragrances, and combinations thereof.
Aspect 45. A method as in aspect 33, wherein said topical composition comprises an additional agent for improving the appearance of at least one sign of aging in skin.
Aspect 46. A method as in aspect 33, wherein said topical composition is substantially free of plant biomass.
Aspect 47. A method of lightening skin, said method comprising applying a topical composition of a non-polar and / or lipophilic extract of *Malva neglecta* to skin in need of lightening to result in lightened skin.
Aspect 48. A method as in aspect 47, wherein said extract of *Malva neglecta* is extracted using a solvent selected from the group consisting of liquid carbon dioxide with or without modifiers, aqueousethanol, C₁-C₈ alcohols, C₁-C₈ alkanes, C₂-C₈ glycols / polyols, C₅-C₈ cycloalkanes, C₁-C₈ alkyl ethers, C₁-C₈ aliphatics, ketones, methylene chloride, ethyl acetate, xylene, toluene, vegetable oil, mineral oil, and combinations thereof.,
Aspect 49. A method as in aspect 48, wherein said solvent is selected from the group consisting ofhexanes, aqueous ethanol, ethanol, methanol, liquid carbon dioxide and combinations thereof.
Aspect 50. A method as in aspect 47, wherein said extract of *Malva neglecta* is extracted using a solvent system comprised of one solvent or combination of solvents having a dielectric constant of a value between about 1.5 and a value of about 40 at 20°C.
Aspect 51. A method as in aspect 50, wherein said solvent is selected from the group consisting of: pentanes, hexanes, heptanes, chloroform, methanol, ethanol, propanols, butanols, glycerine, butylenes glycol, propylene glycol, and liquid carbon dioxide with appropriate polarity modifier, and combinations thereof
Aspect 52. A method as in aspect 47, wherein said extract is of the whole herb of *Malva neglecta.*
Aspect 53. A method as in aspect 47, wherein said topical composition comprises from about 0.001% to about 90% of an extract of *Malva neglecta.*
Aspect 54. A method as in aspect 47, wherein said topical composition comprises from about 0.01% to about 20% by weight of said extract of *Malva neglecta.*
Aspect 55. A method as in aspect 47, wherein said topical composition comprises about 0.01 to about 5% of said extract of *Malva neglecta* herb.
Aspect 56. A method as in aspect 47, wherein said topical composition further comprises a cosmetically acceptable topical carrier.
Aspect 57. A method as in aspect 47, wherein said cosmetically acceptable topical carrier comprises a topical ingredient selected from the group consisting of surfactants, chelating agents, emollients, humectants, conditioners, preservatives, opacifiers, fragrances, and combinations thereof.
Aspect 58. A method as in aspect 47, wherein said topical composition comprises additional skin lightening agents.
Aspect 59. A method as in aspect 47, wherein said topical composition is substantially free of plant biomass.

## Claims

1. A composition of a non-polar, or lipophilic, or non-polar lipophilic extract of *Malva neglecta,* a cosmetically acceptable topical carrier, and an active agent for improving skin barrier function, or for improving the appearance of at least one sign of aging in skin, or for lightening skin.

2. A composition as in claim 1, wherein said composition is substantially free of plant biomass.

3. A composition as in claim 1 or claim 2, wherein said topical composition comprises from about 0.001% to about 90% of an extract of *Malva neglecta.*

4. A composition as in any preceding claim, wherein said topical composition comprises from about 0.01% to about 20% by weight of said extract of *Malva neglecta* herb.

5. A composition as in any preceding claim, wherein said topical composition comprises about 0.01 to about 5% of said extract of *Malva neglecta.*

6. A composition as in any preceding claim, wherein said composition comprises from about 0.01% to about 2% of said extract of *Malva neglecta.*

7. A non-polar and / or lipophilic extract of *Malva neglecta* for use in improving the barrier function of skin, or in improving the appearance of at least one sign of skin aging, or in lightening skin.

8. An extract according to claim 7, wherein said extract of *Malva neglecta* is extracted:
a. using a solvent selected from the group consisting of liquid carbon dioxide with or without modifiers, aqueous ethanol, C₁-C₈ alcohols, C₁-C₈ alkanes, C₂-C₈ glycols/polyols, C₅-C₈ cycloalkanes, C₁-C₈ alkyl ethers, C₁-C₈ aliphatics, ketones, methylene chloride, ethyl acetate, xylene, toluene, vegetable oil, mineral oil, and combinations thereof; e.g. wherein said solvent is selected from the group consisting ofhexanes, aqueous ethanol, ethanol, methanol, liquid carbon dioxide and combinations thereof; or
b. using a solvent system comprised of one solvent or combination of solvents having a dielectric constant of a value between about 1.5 and a value of about 40 at 20°C; e.g. wherein said solvent is selected from the group consisting of: pentanes, hexanes, heptanes, chloroform, methanol, ethanol, propanols, butanols, glycerine, butylenes glycol, propylene glycol, and liquid carbon dioxide with appropriate polarity modifier, and combinations thereof

9. An extract according to claim 7 or claim 8, wherein said extract is of the whole herb of *Malva neglecta.*

10. An extract according to any of claims 7-9, which is formulated in a topical composition for application to skin in need of improving skin barrier function or skin in need of improving the appearance of at least one sign of aging, or skin in need of lightening.

11. An extract according to claim 10, wherein said topical composition comprises from about 0.001% to about 90% of an extract of *Malva neglecta, e.g.* from about 0.01% to about 20% by weight of said extract of *Malva neglecta;* from about 0.01 % to about 5% of said extract of *Malva neglecta;* from about 0.01% to about 2% of said extract of *Malva neglecta.*

12. An extract according to claim 10 or claim 11, wherein said topical composition further comprises a cosmetically acceptable topical carrier, *e.g.* a cosmetically acceptable topical carrier comprising a topical ingredient selected from the group consisting of surfactants, chelating agents, emollients, humectants, conditioners, preservatives, opacifiers, fragrances, and combinations thereof.

13. An extract according to any of claims 10-12, wherein said topical composition comprises additional skin barrier improving agents, or an additional agent for improving the appearance of at least one sign of aging in skin, or additional skin lightening agents.

14. An extract according to any of claims 10-13, wherein said topical composition is substantially free of plant biomass.

15. A cosmetic method of improving the barrier function of skin, said method comprising applying a topical composition of a non-polar and / or lipophilic extract of *Malva neglecta* to skin in need of improving skin barrier function to result in improved skin barrier function.

16. A cosmetic method of improving the appearance of at least one sign of skin aging, said method comprising applying a topical composition of a non-polar and / or lipophilic extract of *Malva neglecta* to skin in need of improving the appearance of at least one sign of aging in skin therein to result in improvement of the appearance of at least one sign of aging in the skin.

17. A cosmetic method of lightening skin, said method comprising applying a topical composition of a non-polar and / or lipophilic extract of *Malva neglecta* to skin in need of lightening to result in lightened skin.

18. The method of any of claims 15-17, wherein the topical composition is that described in any one of claims 10-14
